# EUROPÄISCHE PATENTSCHRIFT

(11) **EP 0 800 657 B1**
(45) Veröffentlichungstag und Bekanntmachung des Hinweises auf die Patenterteilung: **25.11.1998**
(21) Anmeldenummer: 95940936.8
(22) Anmeldetag: 27.12.1995
(51) Int. Cl.: C08F 2/50, C08F 290/04, G02B 1/04, G03F 7/031

(54) **POLYMERE AUF DER GRUNDLAGE VON BLOCKCOPOLYMEREN**
POLYMERS BASED ON BLOCK COPOLYMERS
POLYMERES A BASE DE COPOLYMERES SEQUENCES

(30) Priorität: 30.12.1994 CH 3967/94; 30.12.1994 CH 3968/94
(43) Veröffentlichungstag der Anmeldung: 15.10.1997
(73) Patentinhaber: Novartis AG, 4058 Basel (CH)
(72) Erfinder: CHABRECEK, Peter, Clayton Vic 3169 (AU); LOHMANN, Dieter, CH-4142 Münchenstein (CH); DIETLIKER, Kurt, CH-1700 Fribourg (CH)
(86) Internationale Anmeldenummer: CH9500309
(87) Internationale Veröffentlichungsnummer: WO9621167

(56) Entgegenhaltungen:
- EP-A- 0 281 941
- EP-A- 0 302 831
- EP-A- 0 632 329

## Beschreibung

Die Erfindung betrifft neue Polymere auf der Grundlage von segmentierten Copolymeren, z.B. Blockcopolymeren, die sich insbesondere für die Herstellung von Formkörpern eignen, ferner Formkörper enthaltend derartige Polymere, ferner die Verwendung der Polymere zur Herstellung von Formkörpem und Verfahren zur Herstellung der Polymere und der Formkörper. Bevorzugte Formkörper sind ophthalmische Linsen, insbesondere Kontaktlinsen. Die Polymere zeichnen sich unter anderem dadurch gegenüber bekannten Polymeren aus, dass sie Reste von Photoinitiatoren an den Trennstellen der Blöcke eingebaut enthalten. Die Kopplung der Blöcke erfolgt in einer photochemischen Reaktion, die eine weitgehende Kontrolle der Segmentlänge der aufwachsenden terminalen oder pendenten Polymerblöcke gestattet. Unter einem segmentierten Copolymer werden erfindungsgemäss Blockcopolymere, Pfropfcopolymere, insbesondere Kammcopolymere, oder Sterncopolymere verstanden.

Die erfindungsgemässen segmentierten Copolymere weisen die generelle Formel I auf 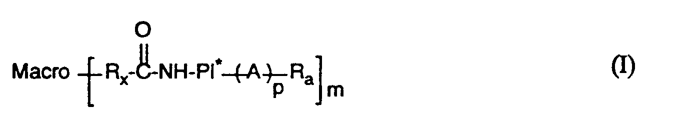 worin Macro für einen m-wertigen Rest eines Makromeren steht, von dem die Anzahl von m Gruppen Rₓ-H entfernt ist,
Rₓ unabhängig voneinander für eine Bindung, -O-, -NR_{N}- oder -S- steht, worin R_{N} für Wasserstoff oder Niederalkyl steht,
PI* für einen zweiwertigen Rest eines Photoinitiators steht,
A für einen bivalenten, substituierten 1,2-Ethylenrest steht, der sich von einem copolymerisierbaren Vinylmonomer dadurch ableitet, dass die Vinyl-Doppelbindung durch eine Einfachbindung ersetzt ist,
jedes Rₐ unabhängig voneinander für eine einwertige Gruppe steht, die geeignet ist, als Kettenabbrecher einer Polymerisation zu dienen,
p unabhängig von m für eine ganze Zahl von 3 bis 500 steht und
m für eine ganze Zahl von 1 bis 100 steht.

Die erfindungsgemässen segmentierten Copolymere der Formel I lassen sich aus den folgenden Bestandteilen aufbauen:
ein Makromer der Formel A:

Macro-(RₓH)ₘ (A)

worin Macro, Rₓ und m wie vorstehend definiert sind aber Rₓ von einer Bindung verschieden ist, zum zweiten um einen Photoinitiator der Formel B,

OCN-PI*-Rₐₐ (B)

worin PI* wie vorstehend definiert ist und Rₐₐ für den Teil eines Photoinititors steht, der bei einer Aufspaltung des Photoinitiators das weniger reaktive Radikal bildet, zum dritten um ein Vinylmonomer, das als Bestandteil "A" in das segmentierte Copolymer eingebaut wird, worin A wie vorstehend definiert ist.

Das erfindungsgemäss geeignete Makromer der Formel A weist eine Anzahl von m Gruppen -RₓH auf, bei denen es sich um Hydroxygruppen (auch um solche, die Bestandteil einer Carboxylgruppe -COOH sind), Aminogruppen oder Niederalkylaminogruppen (auch um solche, die Bestandteil einer Amidgruppe -CONR_{N} sind) oder Mercaptogruppen handelt. Diese Gruppen sind mit der Isocyanatgruppe des Photoinitiators der Formel B coreaktiv. Ein Makromer der Formel A wird geeigneterweise mit m Moläquivalenten des Photoinitiators der Formel B umgesetzt zu einem Makromer der Formel (C)

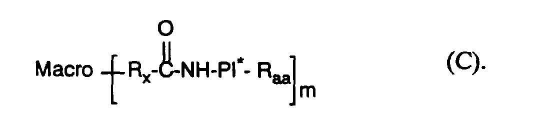

Das so gebildete Makromer der Formel C, das m Photoinitiatoren der Formel B, über eine Brücke -O-CO-NH-, -CO-NH-, -NR_{N}-CO-NH-, -CO-NR_{N}-CO-NH- oder -S-CO-NH- gebunden enthält, wird in einem weiteren Schritt mit p Moläquivalenten eines Vinylmonomeren umgesetzt, das als Bestandteil "A" in das Copolymer der Formel I eingebaut wird. Der Kettenabbruch erfolgt beispielsweise durch das weniger reaktive Radikal des Photoinitiators Rₐₐ der Formel B oder durch andere geeignete Kettenabbrecher, die unter den Reaktionsbedingungen im Reaktionsgemisch vorliegen, wie z.B, H-Radikale oder OH-Radikale oder aus Lösungsmittel gebildete Radikale. Die Variable Rₐ ist bevorzugt der Bestandteil Rₐₐ des Photoinitiators der Formel B.

Die Bedeutung "Bindung" für Rₓ ist nur für den Fall relevant, dass eine Gruppe OH im Makromer als Bestandteil einer COOH-Gruppe vorliegt. Eine COOH-Gruppe reagiert mit einer Isocyanat-Gruppe unter Abspaltung von CO₂ und unter Ausbildung einer Bindung "-CO-NH-". Nur in diesem Fall bedeutet Rₓ eine Bindung im Reaktionsprodukt, nicht jedoch in einem Ausgangsprodukt enthaltend die Gruppe "Rₓ-H".

Der Index p steht vorzugsweise für eine Zahl von 5 bis 200, insbesondere für eine Zahl von 10 bis 100.

Der Index m steht vorzugsweise für eine Zahl von 2 bis 15. Ganz besonders bevorzugt steht der Index m für eine Zahl von 2 bis 5.

Die Gruppen, von denen, je nach Bedeutung des Index m, 1 bis 100 an das Makromer der Formel A gebunden sind, sind entweder endständig oder pendent oder endständig und pendent.

In einer besonders bevorzugten Ausführungsform hat das Makromer der Formel A zwei endständige Gruppen RₓH. Ein hieraus gebildetes erfindungsgemässes segmentiertes Copolymer der Formel I bzw. ein Blockcopolymer der Formel I ist ebenfalls besonders bevorzugt und wird in dieser Erfindung als Triblockcopolymer bezeichnet: Der zentrale Block wird durch das Makromer gebildet, an das zwei Photoinitiatoren gebunden sind, die beiden terminalen Blöcke werden im wesentlichen durch den bivalenten Rest A gebildet.

In einer anderen bevorzugten Ausführungsform weist das Makromer der Formel A nur pendente Gruppen RₓH auf. Ein hieraus gebildetes erfindungsgemässes segmentiertes Copolymer der Formel I bzw. ein Pfropfcopolymer der Formel I ist ebenfalls bevorzugt und wird in dieser Erfindung als Kammpolymer bezeichnet: Der Rücken oder Steg des Kammes wird durch das Makromer gebildet, an das mehrere Photoinitiatoren pendent gebunden sind, die Zinken oder Zähne des Kammes werden im wesentlichen durch die bivalenten Reste A gebildet, die über den Rest des Photoinitiators gebunden sind.

In einer anderen bevorzugten Ausführungsform weist ein cylisches Makromer der Formel A pendente Gruppen RₓH auf. Ein hieraus gebildetes erfindungsgemässes segmentiertes Copolymer der Formel I bzw. ein Pfropfcopolymer der Formel I ist ebenfalls bevorzugt und wird in dieser Erfindung als Stempolymer bezeichnet: Der Mittelpunkt des Sterns wird durch das Makromer gebildet, an das mehrere Photoinitiatoren pendent gebunden sind, die Strahlen des Sternes werden im wesentlichen durch die bivalenten Reste A gebildet, die über den Rest des Photoinitiators gebunden sind.

Es ist bedeutsam, dass alle erfindungsgemässen Copolymere der Formel I und die hieraus erhältlichen vernetzten Polymere sich in ihren Eigenschaften in überraschender Weise von herkömmlichen Copolymeren und Polymeren unterscheiden. Dies gilt zum einen deshalb, weil sich die Kettenlänge der Vinylmonomere (siehe -(A)ₚ- in Formel I) erfindungsgemäss weitgehend kontrollieren lässt. Ferner sind die Copolymere der Formel I überraschenderweise frei oder zumindestens im wesentlichen frei von den Homopolymeren des jeweils verwendeten Vinylmonomeren, wie sie mit anderen in der Literatur beschriebenen radikalischen Makroinitiatoren häufig gebildet werden. Diese vorteilhaften Eigenschaften werden im Zuge der Herstellung der erfindungsgemässen Polymere auf diese übertragen.

So lassen sich die erfindungsgemässen segmentierten Copolymere gezielt zu Folgeprodukten umsetzen bzw. weiterverarbeiten. Insbesondere hervorzuheben ist der Umstand, dass die unvernetzten Copolymere der Formel I sich auf einfache Weise in vernetzte Polymere einbauen lassen, z.B. dadurch dass die Umsetzung einer Verbindung der Formel C mit dem jeweiligen Vinylmonomeren in Gegenwart eines Vernetzers durchgeführt wird. Zusätzlich zu einer derartigen Vernetzung, oder alternativ dazu, können erfindungsgemässe Copolymere der Formel I modifiziert werden, wenn sie im Teil -(A)ₚ- gemäss Formel I reaktive Gruppen aufweisen.

Bei den erfindungsgemässen vernetzten Polymeren handelt es sich daher um die Polymerisationsprodukte eines polymerisationsfähigen Gemisches, das die folgenden Bestandteile enthält:
a) ein Makromer der Formel C 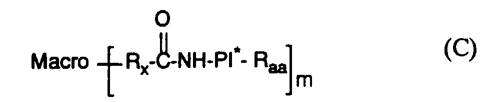 worin Macro für einen m-wertigen Rest eines Makromeren steht, von dem die Anzahl von m Gruppen Rₓ-H entfernt ist,
   Rₓ unabhängig voneinander für eine Bindung, -O-, -NR_{N}- oder -S- steht, worin R_{N} für Wasserstoff oder Niederalkyl steht,
   PI* für einen zweiwertigen Rest eines Photoinitiators steht,
   Rₐₐ für den Teil eines Photoinititors steht, der bei einer Aufspaltung des Photoinitiators das weniger reaktive Radikal bildet, und
   m für eine ganze Zahl von 1 bis 100 steht,
b) ein copolymerisierbares Vinylmonomer und
c) einen Vernetzer.

Weiter handelt es sich bei den erfindungsgemässen Polymeren um die Polymerisationsprodukte, die dadurch erhalten werden, dass man die vorstehend genannten Komponenten a), b) und c) auf an sich bekannte Weise, und insbesondere wie im einzelnen nachstehend beschrieben, miteinander umsetzt.

Ein Makromer der Formel C wird vorzugsweise in einer Menge von 10 bis 90 Gewichtsprozent eingesetzt, insbesondere von 20 bis 80 Gewichtsprozent, ein copolymerisierbares Vinylmonomer wird ebenfalls vorzugsweise in einer Menge von 10 bis 90 Gewichtsprozent, insbesondere von 20 bis 80 Gewichtsprozent, eingesetzt, wobei sich diese Gewichtsprozentangaben auf die Mengen der Komponenten relativ zueinander beziehen. Ein Vemetzer wird vorzugsweise in einer Menge von bis zu 25 Gewichtsprozent, insbesondere in einer Menge von bis zu 12,5 Gewichtsprozent eingesetzt, bezogen auf die Summe der Komponenten a) und b). Diese bevorzugte Gewichtsprozentangabe gilt auch für Vernetzeranteile, die durch nachträgliche Modifizierung eines copolymerisierten Vinylmonomeren erhalten werden.

Bei einem Vernetzer, wie vorstehend als Komponente c) erwähnt, kann es sich um einen typischen copolymerisierbaren oligovinylischen Vernetzer handeln, wie im Stand der Technik bekannt, der dem polymerisationsfähigen Gemisch zugesetzt wird, bevor die Polymerisation zur Herstellung der erfindungsgemässen Polymeren ausgelöst wird.

Alternativ dazu kann es sich bei dem Vernetzer um eine oligofunktionelle Verbindung handeln, die mit im Teil -(A)ₚ- enthaltenen reaktiven Gruppen coreaktiv ist. Unter einer reaktiven Gruppe im -(A)ₚ- Teil wird beispielsweise die OH-Gruppe verstanden, eine hiermit coreaktive Gruppe einer oligofunktionellen Verbindung ist beispielsweise die Isocyanatgruppe, die Carboxylgruppe, auch als Anhydrid, die Epoxygruppe. Geeignete oligofunktionelle Verbindungen sind daher z.B. Diisocyanate, Triisocyanate, Dianhydride, Dicarbonsäuren oder Diepoxide. Eine andere reaktive Gruppe im -(A)ₚ- Teil ist beispielsweise die COOH-Gruppe, hiermit coreaktive Gruppen sind z.B. die Aminogruppe oder die Hydroxygruppe. Geeignete oligofunktionelle Verbindungen sind daher in diesem Fall z.B. Diamine, Diole oder Aminoalkohole. Weitere Beispiele sind dem Fachmann bekannt.

Eine weitere Möglichkeit zur Vernetzung besteht darin, reaktive Gruppen im -(A)ₚ- Teil dahingehend zu modifizieren, dass sie in vernetzbare Gruppen übergeführt werden. Beispiele für derartige Modifizierungen werden nachstehend gegeben.

Bei derartigen reaktiven Gruppen kann es sich beispielsweise um Hydroxygruppen handeln, die von einem Vinylmonomer wie einem Hydroxyniederalkyl(meth)acrylat, beispielsweise 2-Hydroxyethylmethacrylat oder 3-Hydroxypropylmethacrylat, oder von Polyvinylalkohol, stammen, die nachträglich mit einem Vinylisocyanat, wie beispielsweise 2-Isocyanatoethylmethacrylat, umgesetzt werden. Die C-C-Doppelbindungen eines Vinylisocyanates, die auf die so beschriebene Weise eingebaut wurden, gestatten eine Vernetzung zu einem erfindungsgemässen Polymeren und / oder die Copolymerisation mit weiteren Vinylmonomeren oder Divinylmonomeren.

Bei derartigen reaktiven Gruppen kann es sich beispielsweise um Isocyanatgruppen, Carboxylgruppen oder Epoxygruppen handeln, die von einem Vinylisocyanat, einer Vinylcarbonsäure oder einer Vinylepoxyverbindung stammen, wie beispielsweise von 2-Isocyanatoethylmethacrylat, (Meth)acrylsäure oder Glycidyl(meth)acrylat, die nachträglich mit einem Hydroxyniederalkyl(meth)acrylat, beispielsweise 2-Hydroxyethylmethacrylat oder 3-Hydroxypropylmethacrylat umgesetzt werden. Die C-C-Doppelbindungen eines Hydroxyniederalkyl(meth)acrylats, die auf die so beschriebene Weise eingebaut wurden, gestatten eine Vernetzung zu einem erfindungsgemässen Polymeren und / oder die Copolymerisation mit weiteren Vinylmonomeren oder Divinylmonomeren.

Vor- und nachstehend steht eine Formulierung "(meth)acrylat" abkürzend für "methacrylat oder acrylat".

Alle die vorstehend genannten Eigenschaften machen die erfindungsgemässen Polymere geeignet für eine Fülle von Einsatzzwecken als Formkörper verschiedener Art, wie als biomedizinische Materialien, z.B. Implantate, ophthalmische Linsen, insbesondere künstliche Hornhaut, intraokulare Linsen oder, ganz besonders bevorzugt, Kontaktlinsen, oder als medizinische Instrumente, Geräte, Membranen, Drug-Delivery Systeme, oder als Beschichtungen auf anorganischen oder organischen Materialien. Darüberhinaus sind auch die unvernetzten segmentierten Copolymere der Formel I nicht nur als Ausgangsmaterialien für die erfindungsgemässen Polymere, sondern auch hervorragend als Coating-Materialien geeignet. Mit hydrophilen Komponenten "A" entstehen amphiphile Block-, Kamm- oder Sternpolymere, die oberflächenaktive Eigenschaften besitzen und z.B. als Emulgatoren geeignet sind.

Die vorliegende Erfindung betrifft daher Copolymere der Formel I in unvernetzter Form, besonders bevorzugt als Triblockcopolymer, als Kammpolymer oder als Sternpolymer. Die vorliegende Erfindung betrifft ferner vernetzte Polymere wie vorstehend definiert enthaltend die genannten Komponenten als wesentliche oder einzige Komponenten. Die vorliegende Erfindung betrifft ferner Pfropfcopolymere auf der Grundlage von Copolymeren der Formel I, die dadurch modifiziert sind, dass auf im (-Aₚ-)-Teil enthaltene oder dort über reaktive Gruppen eingeführte Vinylgruppen ein Vinylmonomer oder mehrere Vinylmonomere aufgepfropft sind. Die Erfindung betrifft ferner Formkörper, insbesondere Kontaktlinsen aus den genannten Copolymeren, Polymeren oder Pfropfcopolymeren. Die Erfindung betrifft ferner Verfahren zur Herstellung der genannten Copolymere, Polymere oder Pfropfcopolymere unter Verwendung der beschriebenen Ausgangsstoffe und der nachstehenden Verfahrensbedingungen. Die Erfindung betrifft ferner die Herstellung von Formkörpern, insbesondere von Kontaktlinsen, aus den genannten Copolymeren, Polymeren oder Pfropfcopolymeren, sowie die Verwendung der genannten Copolymere, Polymere oder Pfropfcopolymere zur Herstellung von Formkörpern, insbesondere Kontaktlinsen.

Bei den Makromeren der Formel A handelt es sich bevorzugt um Oligomere oder Polymere mit einem mittleren Molekulargewicht von 300 bis 10000 Dalton, und sie enthalten bevorzugt mindestens 3, bevorzugter 3 bis 50 und besonders bevorzugt 5 bis 20 Struktureinheiten. Der Übergang zwischen Oligomeren und Polymeren ist bekannterweise fliessend und nicht genau abzugrenzen. Die Polymeren können 50 bis 10 000, bevorzugter 50 bis 5 000 Struktureinheiten enthalten und ein mittleres Molekulargewicht von 10 000 bis 2 000 000, bevorzugt 10 000 bis 500 000 aufweisen. Die Oligomeren und Polymeren können auch bis zu 95 Mol-%, bevorzugter 5 bis 90 Mol-% comonomere Struktureinheiten ohne H-aktive Gruppen (dieser Terminus ist hier gleichbedeutend mit "RₓH - Gruppen", die wie vorstehend definiert sind, mit der Massgabe, dass Rₓ in diesem Fall von einer Bindung verschieden ist) enthalten, bezogen auf das Polymer.

Bei den Oligomeren und Polymeren mit H-aktiven Gruppen kann es sich um natürliche oder synthetische Oligomere oder Polymere handeln.

Natürliche Oligomere und Polymere sind zum Beispiel Oligo- und Polysaccharide oder deren Derivate, Peptide, Proteine, Glycoproteine, Enzyme und Wachstumsfaktoren. Einige Beispiele sind Cyclodextrine, Stärke, Hyaluronsäure, deacetylierte Hyaluronsäure, Chitosan, Trehalose, Cellobiose, Maltotriose, Maltohexaose, Chitohexaose, Agarose, Chitin 50, Amylose, Glucane, Heparin, Xylan, Pectin, Galactan, poly-Galactosamin, Glycosaminoglycane, Dextran, aminiertes Dextran, Cellulose, Hydroxyalkylcellulosen, Carboxylalkylcellulosen, Heparin, Fucoidan, Chondroitinsulfat, sulfatierte Polysaccharide, Mucopolysaccharide, Gelatine, Casein, Seidenfibroin, Zein, Collagen, Albumin, Globulin, Bilirubin, Ovalbumin, Keratin, Fibronectin und Vitronectin, Pepsin, Trypsin und Lysozym.

Bei den synthetischen Oligomeren und Polymeren kann es sich um die Gruppen -COOH, -OH, -NH₂ oder -NHR_{N} enthaltende Substanzen handeln, wobei R_{N} Niederalkyl, bevorzugt C₁-C₆-Alkyl bedeutet. Es kann sich zum Beispiel um verseifte Polymerisate von Vinylestern oder -ethern (Polyvinylalkohol), hydroxylierte Polydiolefine wie z.B. Polybutadien, Polyisopren oder Chloropren; Polyacrylsäure und Polymethacrylsäure sowie Polyacrylate, Polymethacrylate, Polyacrylamide oder Polymethacrylamide mit Hydroxyalkyl- oder Aminoalkylresten in der Estergruppe oder Amidgruppe; Polysiloxane mit Hydroxyalkyl- oder Aminoalkylgruppen; Polyether aus Epoxiden oder Glycidylverbindungen und Diolen; Polyvinylphenole oder Copolymere von Vinylphenol und olefinischen Comonomeren; sowie Copolymerisate von mindestens einem Monomer aus der Gruppe Vinylalkohol, Vinylpyrrolidon, Acrylsäure, Methacrylsäure, oder Hydroxyalkyl oder Aminoalkyl enthaltenden Acrylaten, Methacrylaten, oder Acrylamid oder Methacrylamid, oder hydroxylierten Dioloefinen mit ethylenisch ungesättigten Comonomeren wie z.B. Acrylnitril, Olefinen, Diolefinen, Vinylchlorid, Vinylidenchlorid, Vinylfluorid, Vinylidenfluorid, Styrol, α-Methylstyrol, Vinylethern und Vinylestem; oder um Polyoxaalkylene mit endständigen OH- oder Aminoalkyloxygruppen handeln.

Bevorzugte Oligomere und Polymere sind zum Beispiel Cyclodextrine mit insgesamt 6 bis 8 einen Ring bildenden Glucosestruktureinheiten oder Hydroxyalkyl- oder Aminoalkylderivaten oder Glucose- oder Maltose-substituierten Derivaten, wovon mindestens eine Struktureinheit der Formel (V) entspricht, 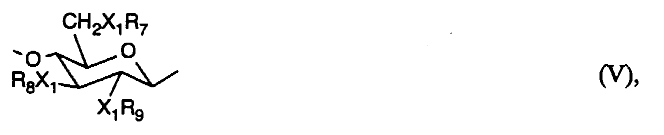 worin R₇, R₈ und R₉ unabhängig voneinander H, C₁-C₄-Alkyl, besonders Methyl, C₂-C₆-Acyl, besonders Acetyl, C₁-C₄-Hydroxyalkyl, besonders Hydroxymethyl oder 2-Hydroxyeth-1-yl, C₂-C₁₀-Aminoalkyl und besonders C₂-C₄-Aminoalkyl, zum Beispiel 2-Aminoeth-1-yl oder 3-Aminoprop-1-yl oder 4-Aminobut-1-yl bedeuten, X₁ für -O- oder -NR_{1B}- steht, wobei pro Cyclodextrineinheit insgesamt 1 bis 10 und bevorzugt 1 bis 6 X₁ -NR_{1B}- bedeuten können und die restlichen X₁ für -O- stehen, wobei R_{1B} Wasserstoff oder Niederalkyl bedeutet.

Andere bevorzugte Oligomere und Polymere sind zum Beispiel Oligo- oder Polysiloxane mit OH- oder NH₂-Gruppen in Alkyl-, Alkoxyalkyl- oder Aminoalkylendgruppen oder -seitenketten. Es kann sich um statistische oder Blockoligomere oder Blockpolymere handeln. Bevorzugtere Oligomere und Polymere sind solche, die
a) 5 bis 100 Mol-% Strukturelemente der Formel (VII) 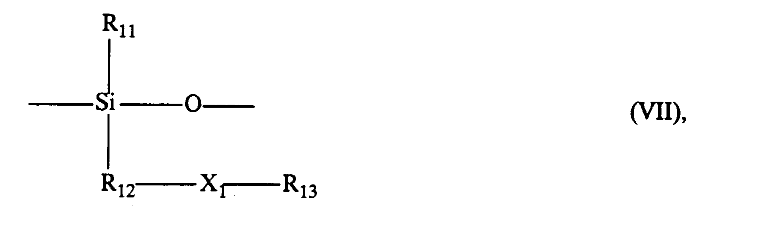 und
b) 95 bis 0 Mol-% Strukturelemente der Formel (VIII) 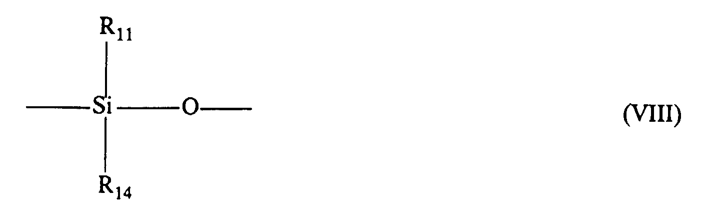 enthalten, bezogen auf das Oligomer oder Polymer, worin R₁₁ gegebenenfalls teilweise oder vollständig mit F substituiertes C₁-C₄-Alkyl, Niederalkenyl, Cyanniederalkyl oder Aryl, bevorzugt Methyl, Ethyl, Vinyl, Allyl, Cyanpropyl oder Trifluormethyl darstellt, R₁₂ C₂-C₆-Alkylen, bevorzugt 1,3-Propylen, -(CH₂)_{z}-(O-CH₂-CHCH₃-)_{z}-, -(CH₂)_{z}-(O-CH₂-CH₂)_{z}- oder -(CH₂)_{z}-NH-(CH₂)_{z}-NH-, bevorzugt -(CH₂)₃-(O-CH₂-CHCH₃-)₂- oder -(CH₂)₃-NH-(CH₂)₂-NH- bedeutet, wobei z eine ganze Zahl von 2 bis 4 darstellt, R₁₄ die Bedeutung von R₁₁ hat oder -R₁₂-X₁-H oder -R₁₂-X₁-R₁₅-H darstellt, X₁ für -O- oder -NH- steht, R₁₃ für einen Rest RₓH steht, und R₁₅ eine direkte Bindung oder eine Gruppe -C(O)-(CHOH)ᵣ-CH₂-O- darstellt, worin r für 0 oder eine ganze Zahl von 1 bis 4 steht.

Bevorzugte oligomere oder polymere Siloxane sind auch solche der Formel (X) 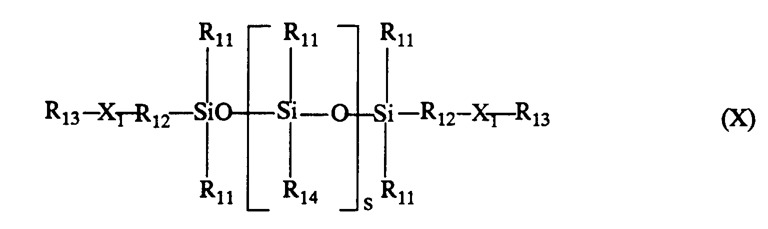 worin R₁₁ gegebenenfalls teilweise oder vollständig mit F substituiertes C₁-C₄-Alkyl, Vinyl, Allyl oder Phenyl, bevorzugt Methyl darstellt, R₁₂ C₂-C₆-Alkylen, bevorzugt 1,3-Propylen bedeutet, R₁₄ die Bedeutung von R₁₁ hat oder -R₁₂-X₁-H oder -R₁₂-X₁-R₁₅-H darstellt, X₁ für -O- oder -NH- steht, s für eine ganze Zahl von 1 - 1000 und bevorzugt von 1 - 150 steht, und R₁₃ für einen Rest RₓH steht, und R₁₅ eine direkte Bindung oder eine Gruppe -C(O)-(CHOH)ᵣ-CH₂-O- darstellt, worin r für 0 oder eine ganze Zahl von 1 bis 4 steht. X₁ steht hier bevorzugt für -NH-.

Andere bevorzugte Oligomere und Polymere sind solche auf der Basis von Oligo- und Polyvinylalkohol. Es kann sich um Homopolymere mit -CH₂CH(OH)-Struktureinheiten oder um Copolymere mit anderen mono- oder bivalenten Struktureinheiten von Olefinen handeln.

Bevorzugter sind solche Oligomere und Polymere, welche
a) 5 bis 100 Mol-% Struktureinheiten der Formel (XI) 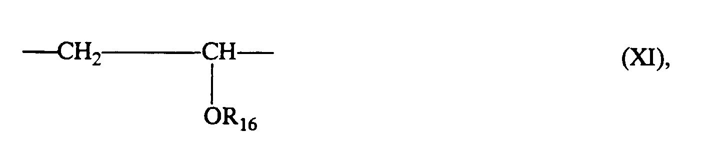 und
b) 95 bis 0 Mol-% Struktureinheiten der Formel (XII) 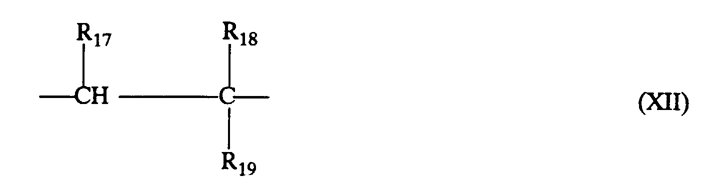 enthalten, worin R₁₆ einen Rest RₓH darstellt, R₁₇ für H, C₁-C₆-Alkyl, -COOR₂₀ oder -COO^{⊖} steht, R₁₈ H, F, Cl, CN oder C₁-C₆-Alkyl bedeutet, und R₁₉ H, OH, R₁₀-H, F, Cl, CN, R₂₀-O-, C₁-C₁₂-Alkyl, -COO^{⊖}, -COOR₂₀, -OCO-R₂₀, Methylphenyl oder Phenyl darstellt, wobei R₁₀ eine direkte Bindung, -(C₁-C₄-Alkylen-O)- oder -(C₂-C₁₀-Alkylen-NH)- darstellt und R₂₀ für C₁-C₁₈-Alkyl, C₅-C₇-Cycloalkyl, (C₁-C₁₂-Alkyl)-C₅-C₇-cycloalkyl, Phenyl, (C₁-C₁₂-Alkyl)phenyl, Benzyl oder (C₁-C₁₂-Alkyl)benzyl steht.

R₁₇ steht bevorzugt für H. Bedeutet R₁₇ Alkyl, so handelt es sich bevorzugt um Methyl oder Ethyl. Bedeutet R₁₇ -COOR₂₀, so stellt R₂₀ bevorzugt C₁-C₁₂-, besonders C₁-C₆-Alkyl dar.

Bedeutet R₁₈ Alkyl, so handelt es sich bevorzugt um C₁-C₄-Alkyl, z.B. Methyl, Ethyl, n-Propyl oder n-Butyl. R₁₈ steht bevorzugt für H, Cl oder C₁-C₄-Alkyl.

Bedeutet R₁₉ die Gruppe R₂₀-O-, so stellt R₂₀ bevorzugt C₁-C₁₂-, besonders C₁-C₆-Alkyl dar. Bedeutet R₁₉ Alkyl, so enthält es bevorzugt 1 bis 6, besonders 1 bis 4 C-Atome. Bedeutet R₁₉ die Gruppe -COOR₂₀, so stellt R₂₀ bevorzugt C₁-C₁₂-, besonders C₁-C₆-Alkyl, Cyclopentyl oder Cyclohexyl dar. Bedeutet R₁₉ die Gruppe -OCO-R₂₀, so stellt R₂₀ bevorzugt C₁-C₁₂-, besonders C₁-C₆-Alkyl, Phenyl oder Benzyl dar.

In einer bevorzugten Ausführungsform stehen R₁₇ für H, R₁₈ für H, F, Cl, Methyl oder Ethyl, und R₁₉ für H, OH, F, Cl, CN, C₁-C₄-Alkyl, C₁-C₆-Alkoxy, C₁-C₆-Hydroxyalkoxy, -COO-C₁-C₆-Alkyl, -OOC-C₁-C₆-Alkyl oder Phenyl.

Besonders bevorzugt sind solche Oligomeren und Polymeren, worin R₁₇ H bedeutet, R₁₈ H oder Methyl darstellt, und R₁₉ H, OH, CN, Methyl, OCH₃, O(CH₂)ₜOH oder -COOCH₃ bedeutet, und t für ganze Zahlen von 2 bis 6 steht.

Eine weitere bevorzugte Gruppe von Oligomeren und Polymeren sind teilweise oder vollständig hydroxyalkylierte Oligo- oder Polyacrylate oder -methacrylate beziehungsweise -acrylamide oder -methacrylamide. Sie können zum Beispiel 5 bis 100 Mol-% Struktureinheiten der Formel (XIII) 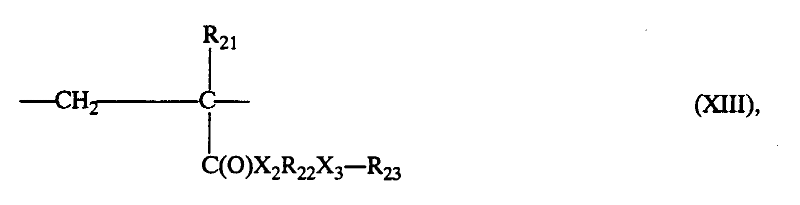 und 95 bis 0 Mol-% Struktureinheiten der Formel (XIV) 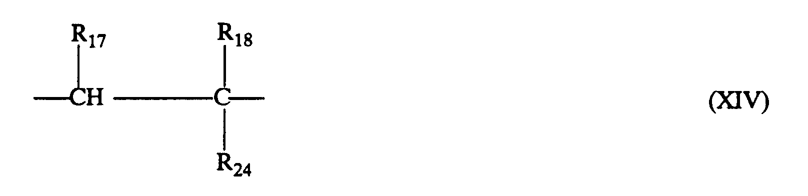 enthalten, worin R₂₁ H oder Methyl bedeutet, X₂ und X₃ unabhängig voneinander -O- oder -NH- darstellen, R₂₂ für -(CH₂)_{c}- steht und c eine ganze Zahl von 2 bis 12, vorzugsweise 2 bis 6 bedeutet, R₂₃ einen Rest der Formel RₓH darstellt, R₁₇ und R₁₈ die zuvor angegebenen Bedeutungen haben, und R₂₄ die gleiche Bedeutung wie R₁₉ hat oder -C(O)X₂R₂₂X₃H bedeutet. Für R₁₇, R₁₈ und R₁₉ gelten die zuvor angegebenen Bevorzugungen. Für X₂ und X₃ gelten die zuvor angegebenen Bevorzugungen.

Andere bevorzugte Oligomere und Polymere sind solche aus Polyalkylenoxiden. Es kann sich zum Beispiel um solche der Formel (XV) mit gleichen oder verschiedenen wiederkehrenden Struktureinheiten -[CH₂CH(R₂₆)-O]- handeln, 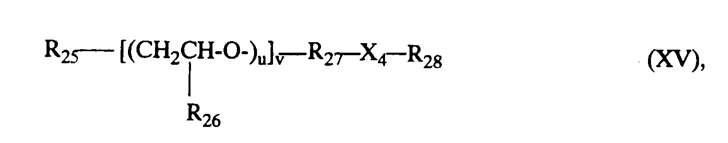 worin R₂₅ die Gruppe R₂₈-X₄- darstellt oder der Rest eines Alkohols oder Polyols mit 1 bis 20 C-Atomen ist, wobei die Wertigkeit dieses Restes von 1 bis v beträgt, R₂₆ H, C₁-C₈-Alkyl, vorzugsweise C₁-C₄-Alkyl und insbesondere bevorzugt Methyl bedeutet, R₂₇ zusammen mit X₄ eine direkte Bindung oder R₂₇ C₂-C₆-Alkylen, vorzugsweise C₃-C₆-Alkylen und insbesondere bevorzugt 1,3-Propylen darstellt, X₄ für -O- oder -NH- steht, R₂₈ einen Rest der Formel RₓH bedeutet, u für einen Zahlenwert von 3 bis 10 000, bevorzugt 5 bis 5 000, besonders bevorzugt 5 bis 1000 und insbesondere bevorzugt 5 bis 100 steht, und v eine ganze Zahl von 1 bis 6, bevorzugt 1 bis 4 bedeutet.

R₂₅ kann der ein- bis vierwertige Rest eines Alkohols oder Polyols sein. Wenn es sich bei R₂₅ um den Rest eines Alkohols handelt, so bedeutet R₂₅ vorzugsweise lineares oder verzweigtes C₃-C₂₀-Alkyl oder -Alkenyl, C₃-C₈- und besonders C₅-C₆-Cycloalkyl, -CH₂-(C₅-C₆-Cycloalkyl), C₆-C₁₀-Aryl und besonders Phenyl und Naphthyl, C₇-C₁₆-Aralkyl und besonders Benzyl und 1-Phenyleth-2-yl. Die cyclischen beziehungsweise aromatischen Reste können mit C₁-C₁₈-Alkyl oder C₁-C₁₈-Alkoxy substituiert sein.

Wenn es sich bei R₂₅ um den Rest eines Diols handelt, so bedeutet R₂₅ vorzugsweise verzweigtes und besonders lineares C₃-C₂₀-Alkylen oder Alkenylen und bevorzugter C₃-C₁₂-Alkylen, C₃-C₈- und besonders C₅-C₆-Cycloalkylen, -CH₂-(C₅-C₆-Cycloalkyl)-, -CH₂-(C₅-C₆-Cycloalkyl)-CH₂-, C₇-C₁₆-Aralkylen und besonders Benzylen, -CH₂-(C₆-C₁₀-Aryl)-CH₂- und besonders Xylylen. Die cyclischen beziehungsweise aromatischen Reste können mit C₁-C₁₂-Alkyl oder C₁-C₁₂-Alkoxy substituiert sein.

Wenn es sich bei R₂₅ um einen dreiwertigen Rest handelt, so leitet sich dieser von aliphatischen oder aromatischen Triolen ab. R₂₅ ist bevorzugt ein dreiwertiger aliphatischer Rest mit 3 bis 12 C-Atomen, der sich besonders von Triolen mit vorzugsweise primären Hydroxylgruppen ableitet. Besonders bevorzugt stellt R₂₅ -CH₂(CH-)CH₂-, HC(CH₂-)₃ oder CH₃C(CH₂-)₃ dar.

Wenn es sich bei R₂₅ um einen vierwertigen Rest handelt, so leitet sich dieser bevorzugt von aliphatischen Tetrolen ab. R₂₅ ist in diesem Fall bevorzugt C(CH₂-)₄.

Bevorzugt steht R₂₅ für einen Rest, der abgeleitet ist von Jeffamine (Texaco), einem Pluriol, einem Poloxamer (BASF) oder Poly(tetramethylenoxid).

Besonders bevorzugt sind Homo- und Blockoligomere und -polymere mit Struktureinheiten der Formeln -[CH₂CH₂-O]- oder -[CH₂CH(CH₃)-O]-.

Geeignet sind auch fluorierte Polyether, die der Formel (XVI) 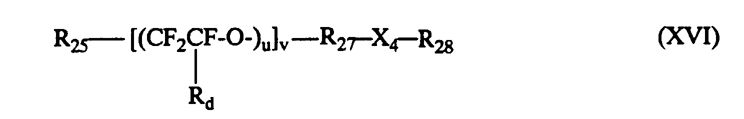 entsprechen, worin R₂₇, R₂₈, X₄, u und v die zuvor angegebenen Bedeutungen haben, R₂₅ die zuvor angegebene Bedeutung hat oder der einwertige Rest eines teil- oder perfluorierten Alkohols mit 1 bis 20, bevorzugt 1 bis 12 und besonders bevorzugt 1 bis 6 C-Atomen, oder der zweiwertige Rest eines teil- oder perfluorierten Diols mit 2 bis 6, bevorzugt 2 bis 4 und besonders bevorzugt 2 oder 3 C-Atomen ist, und R_{d} F oder Perfluoralkyl mit 1 bis 12, bevorzugt 1 bis 6 und besonders bevorzugt 1 bis 4 C-Atomen bedeutet. R_{d} steht besonders bevorzugt für -CF₃.

Weitere geeignete Oligomere und Polymere sind zum Beispiel Polyamine wie Polyvinylamin oder Polyethylenimine. Ebenfalls geeignet ist Poly-ε-lysin.

Als Photoinitiator der Formel B ist grundsätzlich jeder Photoinitiator geeignet, der eine Isocyanatgruppe enthält. Derartige Photoinitiatoren sind beispielsweise in der EP-A-632329 bereits beschrieben. Geeignete Photoinitiatoren weisen üblicherweise das Strukturelement 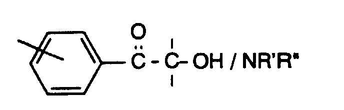 auf (wobei die Formulierung" OH / NR'R" " bedeutet, dass das fragliche Kohlenstoffatom entweder eine OH-Gruppe oder eine NR'R"-Gruppe trägt, worin R' und R" unabhängig voneinander lineares oder verzweigtes Niederalkyl, das mit C₁-C₄-Alkoxy substituiert sein kann, Arylniederalkyl oder Niederalkenyl bedeuten; oder R' und R" zusammen -(CH₂)_{z}-Y₁₁-(CH₂)_{z}- bedeuten, wobei Y₁₁ eine direkte Bindung, -O-, -S-, oder -NR_{1B}- ist und R_{1B} H oder Niederalkyl bedeutet und z eine ganze Zahl von 2 bis 4 bedeutet), das bei geeigneter Anregung zwei Radikale bildet, indem die Bindung zwischen dem Benzoyl - Kohlenstoff und dem sp³ - Kohlenstoff gespalten wird. Ueblicherweise ist das Benzoylradikal das reaktivere, das in aller Regel eine Polymerisation startet. Die Variable PI* aus Formel B entspricht daher vorzugsweise einem derartigen Benzoylradikal. Dieses Benzoylradikal ist, wie im Stand der Technik bekannt, substituiert und enthält erfindungsgemäss zusätzlich eine Isocyanatgruppe. Aus dem vorstehenden ergibt sich, dass das sp³ - Kohlenstoffradikal das weniger reaktive ist, das in der Regel nicht dazu beiträgt, eine Polymerisation zu starten. Stattdessen reagiert es bevorzugt als Kettenabbrecher. Die Variable Rₐₐ aus Formel B entspricht daher vorzugsweise einem derartigen sp³ - Kohlenstoffradikal.

Besonders bevorzugte erfindungsgemässe Photoinitiatoren werden nachstehend beschrieben.

Die erfindungsgemäss verwendeten funktionellen Photoinitiatoren der Formel B sind vorzugsweise Verbindungen der Formel IIa oder IIb 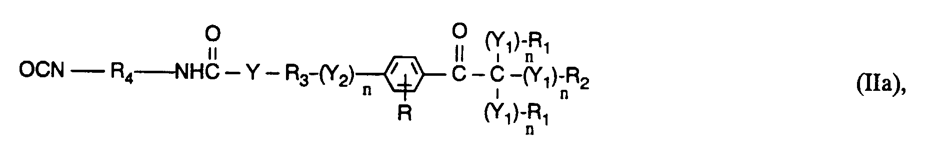 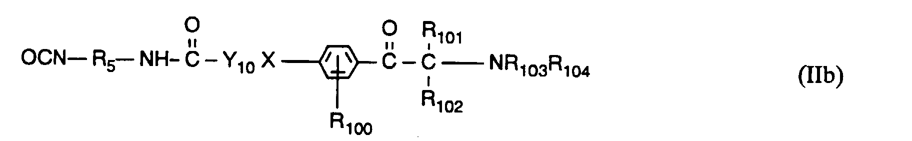 worin Y O, NH oder NR_{1A} bedeutet; Y₁O darstellt; Y₂ für -O-, -O-(O)C-, -C(O)-O- oder -O-C(O)-O- steht; die n unabhängig voneinander für 0 oder 1 stehen; R H, C₁-C₁₂-Alkyl, C₁-C₁₂-Alkoxy oder C₁-C₁₂-AlkylNH- darstellt; die R₁ und R₂ unabhängig voneinander H, lineares oder verzweigtes C₁-C₈-Alkyl, C₁-C₈-Hydroxyalkyl oder C₆-C₁₀-Aryl darstellen, oder zwei Gruppen R₁-(Y₁)ₙ- zusammen -(CH₂)ₓ- bedeuten, oder die Gruppen R₁-(Y₁)ₙ- und R₂-(Y₁)ₙ- zusammen einen Rest der Formel 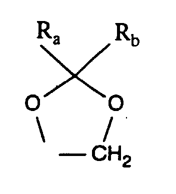 bilden; R₃ eine direkte Bindung oder lineares oder verzweigtes C₁-C₈-Alkylen darstellt, das unsubstituiert oder mit -OH substituiert ist und/oder gegebenenfalls mit ein oder mehreren Gruppen -O-, -O-C(O)- oder -O-C(O)-O- unterbrochen ist; R₄ verzweigtes C₃-C₁₈-Alkylen, unsubstituiertes oder mit C₁-C₄-Alkyl oder C₁-C₄-Alkoxy substituiertes C₆-C₁₀-Arylen, oder unsubstituiertes oder mit C₁-C₄-Alkyl oder C₁-C₄-Alkoxy substituiertes C₇-C₁₈-Aralkylen, unsubstituiertes oder mit C₁-C₄-Alkyl oder C₁-C₄-Alkoxy substituiertes C₃-C₈-Cycloalkylen, unsubstituiertes oder mit C₁-C₄-Alkyl oder C₁-C₄-Alkoxy substituiertes C₃-C₈-Cycloalkylen-C_{y}H_{2y}- oder unsubstituiertes oder mit C₁-C₄-Alkyl oder C₁-C₄-Alkoxy substituiertes -C_{y}H_{2y}-(C₃-C₈-Cycloalkylen)-C_{y}H_{2y}- bedeutet; R₅ unabhängig die gleiche Bedeutung wie R₄ hat oder lineares C₃-C₁₈-Alkylen darstellt; R_{1A} für Niederalkyl steht; x ganze Zahlen von 3 bis 5 bedeutet; y ganze Zahlen von 1 bis 6 bedeutet; Rₐ und R_{b} unabhängig voneinander H, C₁-C₈-Alkyl, C₃-C₈-Cycloalkyl, Benzyl oder Phenyl darstellen; mit den Massgaben, dass n in den Gruppen -(Y₁)ₙ-R₁ für 0 steht, wenn R₂ H bedeutet; dass höchstens zwei Y₁ der -(Y₁)ₙ-Gruppen O bedeuten sowie n in den anderen -(Y₁)ₙ-Gruppen für 0 steht; und dass n in der Gruppe -(Y₂)ₙ- für 0 steht, wenn R₃ eine direkte Bindung bedeutet; und worin ferner X bivalentes -O-, -NH-, -S-, Niederalkylen oder 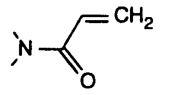 bedeutet; Y₁₀-O-(CH₂)_{y}- oder eine direkte Bindung darstellt, wobei y ganze Zahlen von 1 - 6 bedeutet und dessen endständige CH₂-Gruppe mit dem benachbarten X in Formel (IIb) verknüpft ist; R₁₀₀ H, C₁-C₁₂-Alkyl, C₁-C₁₂-Alkoxy, C₁-C₁₂-AlkylNH- oder -NR_{1A}R_{1B} darstellt, wobei R_{1A} für Niederalkyl und R_{1B} für H oder Niederalkyl steht; R₁₀₁ für lineares oder verzweigtes Niederalkyl, Niederalkenyl oder Arylniederalkyl steht; R₁₀₂ unabhängig von R₁₀₁ die gleiche Bedeutung wie R₁₀₁ hat oder Aryl bedeutet, oder R₁₀₁ und R₁₀₂ zusammen -(CH₂)ₘ- bedeuten, wobei m ganze Zahlen von 2 - 6 bedeutet; R₁₀₃ und R₁₀₄ unabhängig voneinander lineares oder verzweigtes Niederalkyl, das mit C₁-C₄-Alkoxy substituiert sein kann, Arylniederalkyl oder Niederalkenyl bedeuten; oder R₁₀₃ und R₁₀₄ zusammen -(CH₂)_{z}-Y₁₁-(CH₂)_{z}- bedeuten, wobei Y₁₁ eine direkte Bindung, -O-, -S-, oder -NR_{1B}- ist und R_{1B} H oder Niederalkyl bedeutet und z eine ganze Zahl von 2 bis 4 bedeutet.

In einer bevorzugten Ausführungsform steht Y für O.

R_{1A} als Alkyl kann zum Beispiel Methyl, Ethyl, n- oder i-Propyl, n-, i- oder t-Butyl, Pentyl oder Hexyl sein. Bevorzugt stellt R_{1A} Methyl dar.

Die Gruppe R enthält als Alkyl, Alkoxy oder AlkylNH- bevorzugt 1 bis 6 und besonders bevorzugt 1 bis 4 C-Atome. Einige Beispiele sind Methyl, Ethyl, n- oder i-Propyl, n-, 1-oder t-Butyl, Pentyl, Hexyl, Octyl, Decyl, Dodecyl, Methoxy, Ethoxy, Propoxy, Butoxy, und MethylNH-. Insbesondere bevorzugt steht R für H.

R₁ ist als Alkyl bevorzugt linear und enthält bevorzugt 1 bis 4 C-Atome. Einige Beispiele sind Methyl, Ethyl, n- oder i-Propyl, n-, i- oder t-Butyl, Pentyl, Hexyl, Heptyl und Octyl. Besonders bevorzugt handelt es sich bei R₁ um Methyl oder Ethyl. R₁ kann als Aryl zum Beispiel Naphthyl und besonders Phenyl bedeuten. Wenn beide Gruppen R₁-(Y₁)ₙ-zusammen für -(CH₂)ₓ- stehen, ist x bevorzugt 4 und besonders bevorzugt 5. R₁ ist als Hydroxyalkyl bevorzugt linear und enthält bevorzugt 1 bis 4 C-Atome. Einige Beispiele sind Hydroxymethyl und 2-Hydroxyeth-1-yl.

Für R₂ gelten die gleichen Bevorzugungen wie für R₁. Besonders bevorzugt steht R₂ für H, Methyl oder Ethyl.

Rₐ und R_{b} bedeuten bevorzugt unabhängig voneinander H oder C₁-C₄-Alkyl, zum Beispiel Methyl oder Ethyl.

In einer bevorzugten Untergruppe bedeutet R₁ bevorzugt Ethyl und besonders bevorzugt Methyl oder die beiden Gruppen R₁-(Y₁)ₙ- zusammen Pentamethylen, steht n in der Gruppe -(Y₁)ₙ-R₂ bevorzugt für 0, stellt R₂ bevorzugt Methyl, Hydroxmethyl oder H dar und steht R für H.

In einer anderen bevorzugten Ausführungsform stehen in der Gruppe -(Y₁)ₙ-R₂ Y₁für O, n für 1 und R₂ für H. Insbesondere steht in diesem Fall n in den Gruppen R₁-(Y₁)ₙ- für 0.

R₃ enthält als Alkylen bevorzugt 1 bis 6 und besonders bevorzugt 1 bis 4 C-Atome und bevorzugt ist das Alkylen linear. Einige Beispiele sind Methylen, Ethylen, 1,2- oder 1,3-Propylen, 1,2-, 1,3- oder 1,4-Butylen, Pentylen, Hexylen, Heptylen und Octylen. Bevorzugt sind Methylen, Ethylen, 1,3-Propylen und 1,4-Butylen. Ganz besonders bevorzugt stellt R₃ Ethylen dar; oder eine direkte Bindung, wobei n in der Gruppe -(Y₂)ₙ- für 0 steht.

Bei R₃ als mit Hydroxy substituiertem Alkylen kann es sich zum Beispiel insbesondere um 2-Hydroxy-1,3-propylen oder auch um 2-Hydroxy-1,3- oder-1,4-butylen handeln. Mit -O- unterbrochenes und gegebenenfalls mit -OH substituiertes Alkylen ist zum Beispiel -CH₂CH₂-O-CH₂CH₂-, -CH₂CH₂-O-CH₂CH₂-O-CH₂CH₂-, -CH₂CH₂-O-CH₂CH₂-O-CH₂CH₂-O-CH₂CH₂-, [-CH(CH₃)CH₂-O-CH(CH₃)CH₂-], -CH(CH₃)CH₂-O-CH₂CH₂-, -CH(C₂H₅)CH₂-O-CH₂CH₂-, [-CH(C₂H₅)CH₂-O-CH(C₂H₅)CH₂-] oder -CH₂CH₂CH₂CH₂-O-CH₂CH₂CH₂CH₂- und -CH₂CH(OH)CH₂-O-CH₂CH₂-. Mit -O-C(O)- oder -C(O)-O- unterbrochenes Alkylen ist zum Beispiel -CH₂CH₂-C(O)-O-CH₂- oder -CH₂CH₂-O-C(O)-CH₂-. Mit -O-C(O)-O- unterbrochenes Alkylen ist zum Beispiel -CH₂CH₂-O-C(O)O-CH₂CH₂- oder -CH₂CH₂-O-C(O)-O-CH₂-.

Bei den Substituenten C₁-C₄-Alkyl und C₁-C₄-Alkoxy handelt es sich vorzugsweise um Methyl, Ethyl, Methoxy oder Ethoxy.

R₄ enthält als verzweigtes Alkylen bevorzugt 3 bis 14 und besonders bevorzugt 4 bis 10 C-Atome. Beispiele für Alkylen sind 1,2-Propylen, 2-Methyl- oder 2,2-Dimethyl-1,3-propylen, 1,2-, 1,3- und 2,3-Butylen, 2-Methyl- oder 2,3-Dimethyl-1,4-butylen, 1,2-, 1,3-oder 1,4-Pentylen, 2-Methyl- oder 3-Methyl- oder 4-Methyl- oder 2,3-Dimethyl- oder 2,4-Dimethyl oder 3,4-Dimethyl- oder 2,3,4-Trimethyl oder 2,2,3-Trimethyl- 2,2,4-Trimethyl- oder 2,2,3,3-Tetramethyl- oder 2,2,3,4-Tetramethyl-1,5-pentylen, 1,2-, 1,3-, 1,4- oder 1,5-Hexylen, 2-Methyl- oder 3-Methyl oder 4-Methyl- oder 2,2-Dimethyl- oder 3,3-Dimethyl- oder 2,3-Dimethyl- oder 2,4-Dimethyl- oder 3,4-Dimethyl- oder 2,2,3-Trimethyl- oder 2,2,4-Trimethyl- oder 2,2,5-Trimethyl- oder 2,3,4-Trimethyl- oder 2,2,4,5-Tetramethyl-1,6-hexylen. Weitere Beispiele- sind in der EP-A-632329 offenbart.

Einige bevorzugte verzweigte Alkylenreste sind 2,2-Dimethyl-1,4-butylen, 2,2-Dimethyl-1,5-pentylen, 2,2,3- oder 2,2,4-trimethyl-1,5-pentylen, 2,2-Dimethyl-1,6-hexylen, 2,2,3-oder 2,2,4- oder 2,2,5-Trimethyl-1,6-hexylen, 2,2-Dimethyl-1,7-heptylen, 2,2,3- oder 2,2,4- oder 2,2,5- oder 2,2,6-Trimethyl-1,7-heptylen, 2,2-Dimethyl-1,8-octylen, 2,2,3-oder 2,2,4- oder 2,2,5- oder 2,2,6- oder 2,2,7-Trimethyl-1,8-octylen.

Wenn R₄ Arylen ist, handelt es sich bevorzugt um Naphthylen und besonders bevorzugt um Phenylen. Wenn das Arylen substituiert ist, befindet sich ein Substituent vorzugsweise in Orthostellung zu einer Isocyanatgruppe. Beispiele für substituiertes Arylen sind 1-Methyl-2,4-phenylen, 1,5-Dimethyl-2,4-phenylen, 1-Methoxy-2,4-phenylen und 1-Methyl-2,7-naphthylen.

R₄ als Aralkylen ist bevorzugt Naphthylalkylen und besonders bevorzugt Phenylalkylen. Die Alkylengruppe im Aralkylen enthält bevorzugt 1 bis 12, besonders bevorzugt 1 bis 6 und insbesondere bevorzugt 1 bis 4 C-Atome. Ganz besonders bevorzugt stellt die Alkylengruppe im Aralkylen Methylen oder Ethylen dar. Einige Beispiele sind 1,3- oder 1,4-Benzylen, Naphth-2-yl-7-methylen, 6-Methyl-1,3- oder 1,4-benzylen, 6-Methoxy-1,3- oder 1,4-benzylen.

Wenn R₄ Cycloalkylen ist, handelt es sich bevorzugt um C₅- oder C₆-Cycloalkylen, das unsubstituiert oder mit Methyl substituiert ist. Einige Beispiele sind 1,3-Cyclobutylen, 1,3-Cyclopentylen, 1,3- oder 1,4-Cyclohexylen, 1,3- oder 1,4-Cycloheptylen, 1,3- oder 1,4- oder 1,5-Cyclooctylen, 4-Methyl-1,3-Cyclopentylen, 4-Methyl-1,3-Cyclohexylen, 4,4-Dimethyl-1,3-Cyclohexylen, 3-Methyl- oder 3,3-Dimethyl-1,4-Cyclohexylen, 3,5-Dimethyl-1,3-Cyclohexylen, 2,4-Dimethyl-1,4-Cyclohexylen.

Wenn R₄ Cycloalkylen-C_{y}H_{2y}- bedeutet, handelt es sich bevorzugt um Cyclopentylen-C_{y}H_{2y}- und besonders um Cyclohexylen-C_{y}H_{2y}-, das unsubstituiert oder mit vorzugsweise 1 bis 3 C₁-C₄-Alkyl, besonders bevorzugt Methyl, substituiert ist. In der Gruppe -C_{y}H_{2y}- steht y bevorzugt für ganze Zahlen von 1 bis 4. Bevorzugter stellt die Gruppe -C_{y}H_{2y}- Ethylen und besonders bevorzugt Methylen dar. Einige Beispiele sind Cyclopent-1-yl-3-methylen, 3-Methyl-cyclopent-1-yl-3-methylen, 3,4-Dimethyl-cyclopent-1-yl-3-methylen, 3,4,4-Trimethyl-cyclopent-1-yl-3-methylen, Cyclohex-1-yl-3- oder -4-methylen, 3- oder 4- oder 5-Methyl-cyclohex-1-yl-3- oder -4-methylen, 3,4- oder 3,5-Dimethyl-cyclohex-1-yl-3- oder -4-methylen, 3,4,5- oder 3,4,4- oder 3,5,5-Trirnethyl-cyclohex-1-yl-3- oder -4-methylen.

Wenn R₄-C_{y}H_{2y}-Cycloalkylen-C_{y}H_{2y}- bedeutet, handelt es sich bevorzugt um -C_{y}H_{2y}-Cyclopentylen-C_{y}H_{2y}- und besonders um -C_{y}H_{2y}-Cyclohexylen-C_{y}H_{2y}-, das unsubstituiert oder mit vorzugsweise 1 bis 3 C₁-C₄-Alkyl, besonders bevorzugt Methyl, substituiert ist. In der Gruppe -C_{y}H_{2y}- steht y bevorzugt für ganze Zahlen von 1 bis 4. Bevorzugter stellen die Gruppen -C_{y}H_{2y}- Ethylen und besonders bevorzugt Methylen dar. Einige Beispiele sind Cyclopentan-1,3-dimethylen, 3-Methyl-cyclopentan-1,3-dimethylen, 3,4-Dimethylcyclopentan-1,3-dimethylen, 3,4,4-Trimethyl-cyclopentan-1,3-dimethylen, Cyclohexan-1,3- oder -1,4-dimethylen, 3- oder 4- oder 5-Methyl-cyclohexan-1,3- oder -1,4-dimethylen, 3,4- oder 3,5-Dimethyl-cyclohexan-1,3- oder -1,4-dimethylen, 3,4,5- oder 3,4,4- oder 3,5,5-Trimethyl-cyclohexan-1,3- oder -1,4-dimethylen.

Wenn R₅ die gleiche Bedeutung wie R₄ hat, gelten auch die zuvor für R₄ angegebenen Bevorzugungen. R₅ enthält als lineares Alkylen bevorzugt 3 bis 12 und besonders bevorzugt 3 bis 8 C-Atome. Einige Beispiele für lineares Alkylen sind 1,3-Propylen, 1,4-Butylen, 1,5-Pentylen, 1,6-Hexylen, 1,7-Heptylen, 1,8-Octylen, 1,9-Nonylen, 1,10-Decylen, 1,11-Undecylen, 1,12-Dodecylen, 1,14-Tetradecylen und 1,18-Octadecylen.

Eine bevorzugte Bedeutung von X ist -O-, -NH-, -S- oder Niederalkylen. Stärker bevorzugt steht X für -O- oder -S- und besonders bevorzugt für -O-.

In einer bevorzugten Bedeutung von Y₁₀ steht der Index y für 1- 5, stärker bevorzugt für 2 - 4 und ausserordentlich bevorzugt für 2 - 3, so dass Y₁₀ zum Beispiel Ethylenoxy oder Propylenoxy bedeutet. In einer weiteren bevorzugten Bedeutung steht Y₁₀ für eine direkte Bindung, wobei X dann bevorzugt mindestens ein Heteroatom darstellt oder enthält.

Die Gruppe R₁₀₀ enthält als Alkyl, Alkoxy, AlkylNH- oder -NR_{1A}R_{1B} bevorzugt 1 bis 6 und besonders bevorzugt 1 bis 4 C-Atome. Einige Beispiele sind Methyl, Ethyl, n- oder i-Propyl, n-, i- oder t-Butyl, Pentyl, Hexyl, Octyl, Decyl, Dodecyl, Methoxy, Ethoxy, Propoxy, Butoxy, N,N-Dimethylamino und N-Methylamino. Insbesondere bevorzugt steht R für H. Eine bevorzugte Bedeutung von -NR_{1A}R_{1B} ist N,N-Dimethylamino, N-Methylamino, N-Methyl-N-Ethylamino, N-Ethylamino, N,N-Diethylamino, N-Isopropylamino oder N,N-Diisopropylamino.

R₁₀₁ bedeutet bevorzugt Allyl, Benzyl, lineares C₁-C₄-Alkyl wie zum Beispiel Methyl oder Ethyl.

R₁₀₂ hat bevorzugt die gleiche Bedeutung wie R₁₀₁, ist stärker bevorzugt lineares Niederalkyl mit 1 bis 4 C-Atomen und besonders bevorzugt 1 bis 2 C-Atomen. R₁₀₂ kann als Aryl zum Beispiel Naphthyl oder besonders Phenyl bedeuten, das unsubstituiert oder durch Niederalkyl oder Niederalkoxy substituiert ist. Wenn R₁₀₁ und R₁₀₂ zusammen für -(CH₂)ₘ- stehen, ist m bevorzugt 4 oder 5 und besonders bevorzugt 5.

R₁₀₃ steht bevorzugt für lineares Niederalkyl mit 1 bis 4 C-Atomen, Benzyl oder Allyl, und stärker bevorzugt für Methyl oder Ethyl.

R₁₀₄ steht bevorzugt für lineares Niederalkyl mit 1 bis 4 C-Atomen, und stärker bevorzugt für Methyl oder Ethyl.

Wenn R₁₀₃ und R₁₀₄ zusammen -(CH₂)_{z}-Y₁₁-(CH₂)_{z}- bedeuten ist Y₁₁ bevorzugt eine direkte Bindung, -O- oder -N(CH₃)- und ganz besonders -O-; z ist bevorzugt 2 - 3 und besonders bevorzugt 2.

Eine bevorzugte Untergruppe von Verbindungen der Formel IIa sind solche, worin in den Gruppen R₁-(Y₁)ₙ- n für 0 steht, Y, Y₂ und Y₁in der Gruppe R₂-(Y₁)ₙ- je O bedeuten, n in der Gruppe R₂-(Y₁)ₙ- für 0 oder 1 steht, R₁ C₁-C₄-Alkyl oder Phenyl bedeuten oder die Gruppen R₁-(Y₁)ₙ- zusammen Tetramethylen oder Pentamethylen darstellen, R₂ C₁-C₄-Alkyl oder H darstellt, R Wasserstoff bedeutet, n in der Gruppe -(Y₂)-ₙ für 0 oder 1 steht, R₃ lineares oder verzweigtes C₂-C₄-Alkylen darstellt oder eine direkte Bindung bedeutet, wobei n in der Gruppe -(Y₂)-ₙ für 0 steht, R₄ verzweigtes C₅-C₁₀-Alkylen, Phenylen oder mit 1 bis 3 Methylgruppen substituiertes Phenylen, Benzylen oder mit 1 bis 3 Methylgruppen substituiertes Benzylen, Cyclohexylen oder mit 1 bis 3 Methylgruppen substituiertes Cyclohexylen, Cyclohexyl-C_{y}H_{2y}- oder -C_{y}H_{2y}-Cyclohexyl-C_{y}H_{2y}- oder mit 1 bis 3 Methylgruppen substituiertes Cyclohexyl-C_{y}H_{2y}- oder -C_{y}H_{2y}-Clyclohexyl-C_{y}H_{2y}-bedeutet, R₅ die für R₄ angegebenen Bedeutungen hat oder lineares C₃-C₁₀-Alkylen darstellt, und y für 1 oder 2 steht.

Eine besonders bevorzugte Untergruppe von Verbindungen der Formel IIa sind solche, worin in den Gruppen R₁-(Y₁)ₙ- und -(Y₂)-ₙ n für 0 steht, Y, Y₂ und Y₁in der Gruppe R₂-(Y₁)ₙ- je O bedeuten, n in der Gruppe R₂-(Y₁)ₙ- für 0 oder 1 steht, R₁ Methyl oder Phenyl bedeutet oder die Gruppen R₁-(Y₁)ₙ- zusammen Pentamethylen darstellen, R₂ Methyl oder H darstellt, R Wasserstoff bedeutet, n in der Gruppe -(Y₂)-ₙ für 1 steht und R₃ Ethylen darstellt oder n in der Gruppe -(Y₂)-ₙ für 0 steht und R₃ eine direkte Bindung bedeutet, R₄ verzweigtes C₆-C₁₀-Alkylen, Phenylen oder mit 1 bis 3 Methylgruppen substituiertes Phenylen, Benzylen oder mit 1 bis 3 Methylgruppen substituiertes Benzylen, Cyclohexylen oder mit 1 bis 3 Methylgruppen substituiertes Cyclohexylen, Cyclohexyl-CH₂- oder mit 1 bis 3 Methylgruppen substituiertes Cyclohexyl-CH₂- bedeutet, und R₅ die für R₄ angegebenen Bedeutungen hat oder lineares C₅-C₁₀-Alkylen darstellt.

Eine bevorzugte Untergruppe von Verbindungen der Formel IIb sind solche, worin R₁₀₁ für lineares Niederalkyl, Niederalkenyl oder Arylniederalkyl steht; R₁₀₂ unabhängig von R₁₀₁ die gleiche Bedeutung wie R₁₀₁ hat oder Aryl bedeutet; R₁₀₃ und R₁₀₄ unabhängig voneinander lineares oder verzweigtes Niederalkyl, das mit C₁-C₄-Alkoxy substituiert sein kann, Arylniederalkyl oder Niederalkenyl bedeuten; oder R₁₀₃ und R₁₀₄ zusammen -(CH₂)_{z}-Y₁₁-(CH₂)_{z}- bedeuten, wobei Y₁₁ eine direkte Bindung, -O-, -S-, oder -NR_{1B}- ist und R_{1B} H oder Niederalkyl bedeutet und z eine ganze Zahl von 2 bis 4 bedeutet; und R₅ lineares oder verzweigtes C₃-C₁₈-Alkylen, unsubstituiertes oder mit C₁-C₄-Alkyl oder C₁-C₄-Alkoxy substituiertes C₆-C₁₀-Arylen, oder unsubstituiertes oder mit C₁-C₄-Alkyl oder C₁-C₄-Alkoxy substituiertes C₇-C₁₈-AralkyIen, unsubstituiertes oder mit C₁-C₄-Alkyl oder C₁-C₄-Alkoxy substituiertes C₁₃-C₂₄-Arylenalkylenarylen, unsubstituiertes oder mit C₁-C₄-Alkyl oder C₁-C₄-Alkoxy substituiertes C₃-C₈-Cycloalkylen, unsubstituiertes oder mit C₁-C₄-Alkyl oder C₁-C₄-Alkoxy substituiertes C₃-C₈-Cycloalkylen-C_{y}H_{2y}- oder unsubstituiertes oder mit C₁-C₄-Alkyl oder C₁-C₄-Alkoxy substituiertes -C_{y}H_{2y}-(C₃-C₈-Cycloalkylen)-C_{y}H_{2y}- bedeutet, wobei y eine ganze Zahl von 1 - 6 bedeutet.

Eine bevorzugte Untergruppe von Verbindungen der Formel IIb sind solche, worin X bivalentes -O-, -NH-, -S- oder -(CH₂)_{y}- bedeutet; Y₁₀-O-(CH₂)_{y}- oder eine direkte Bindung darstellt, wobei y ganze Zahlen von 1 - 6 bedeutet und dessen endständige CH₂-Gruppe mit dem benachbarten X in Formel (IIb) verknüpft ist; R₁₀₀ H, C₁-C₁₂-Alkyl oder C₁-C₁₂-Alkoxy darstellt; R₁₀₁ für lineares Niederalkyl, Niederalkenyl oder Arylniederalkyl steht; R₁₀₂ unabhängig von R₁₀₁ die gleiche Bedeutung wie R₁₀₁ hat oder Aryl bedeutet, oder R₁₀₁ und R₁₀₂ zusammen -(CH₂)ₘ- bedeuten, wobei m ganze Zahlen von 2 - 6 bedeutet; R₁₀₃ und R₁₀₄ unabhängig voneinander lineares oder verzweigtes Niederalkyl, das mit C₁-C₄-Alkoxy substituiert sein kann, Arylniederalkyl oder Niederalkenyl bedeuten; oder R₁₀₃ und R₁₀₄ zusammen -(CH₂)_{z}-Y₁₁-(CH₂)_{z}- bedeuten, wobei Y₁₁ eine direkte Bindung, -O-, -S-, oder -NR_{1B}- ist und R_{1B} H oder Niederalkyl bedeutet und z eine ganze Zahl von 2 bis 4 bedeutet; und R₅ verzweigtes C₆-C₁₀-Alkylen, Phenylen oder mit 1 bis 3 Methylgruppen substituiertes Phenylen, Benzylen oder mit 1 bis 3 Methylgruppen substituiertes Benzylen, Cyclohexylen oder mit 1 bis 3 Methylgruppen substituiertes Cyclohexylen, Cyclohexylen-CH₂- oder mit 1 bis 3 Methylgruppen substituiertes Cyclohexylen-CH₂- bedeutet.

Eine besonders bevorzugte Untergruppe von Verbindungen der Formel IIb sind solche, worin R₁₀₁ Methyl, Allyl, Toluylmethyl oder Benzyl bedeutet, R₁₀₂ Methyl, Ethyl, Benzyl oder Phenyl bedeutet oder R₁₀₁ und R₁₀₂ zusammen Pentamethylen darstellen, R₁₀₃ und R₁₀₄ unabhängig voneinander für Niederalkyl mit bis zu 4 C-Atomen stehen oder R₁₀₃ und R₁₀₄ zusammen für -CH₂CH₂OCH₂CH₂- stehen, und R₅ verzweigtes C₆-C₁₀-Alkylen, Phenylen oder mit 1 bis 3 Methylgruppen substituiertes Phenylen, Benzylen oder mit 1 bis 3 Methylgruppen substituiertes Benzylen, Cyclohexylen oder mit 1 bis 3 Methylgruppen substituiertes Cyclohexylen, Cyclohexylen-CH₂- oder mit 1 bis 3 Methylgruppen substituiertes Cyclohexylen-CH₂- bedeutet.

Bei den Gruppen R₄ und R₅ handelt es sich insbesondere um solche, die die Reaktivität der OCN-Gruppe vermindern, was im wesentlichen durch eine sterische Hinderung oder elektronische Einflüsse an mindestens einem benachbarten C-Atom erreicht wird. Bevorzugt sind R₄ und R₅ daher unter anderem unsymmetrische Reste, z.B. in α- oder besonders β-Stellung zur OCN-Gruppe verzweigtes Alkylen, oder in mindestens einer α-Stellung wie definiert substituierte cyclische Kohlenwasserstoffreste.

Unter einem copolymerisierbaren Vinylmonomer wird im Rahmen dieser Erfindung insbesondere ein Monomer verstanden, das eine Vinylgruppe enthält und bereits im Zusammenhang mit Copolymerisaten, die für Kontaktlinsen Verwendung gefunden haben, erwähnt wurde. Unter einer Vinylgruppe wird in diesem Zusammenhang nicht ausschliesslich die Vinylgruppierung "-CH=CH₂" verstanden, sondern allgemein jede Gruppierung, die eine Kohlenstoff-Kohlenstoff-Doppelbindung aufweist. Speziell bevorzugte Bedeutungen des Wortbestandteils "Vinyl" bei Vinylmonomeren werden aus den nachstehenden Erläuterungen im Zusammenhang mit Verbindungen der Formel III deutlich. Copolymerisierbare Vinylmonomere im Sinne dieser Erfindung sind beispielsweise in den EP-A-374,752, EP-A-417,235 und in der EP-A-455,587 bereits offenbart worden.

Insbesondere sind die Monomere, von denen man ausgeht, um den Bestandteil A der Formel I für die erfindungsgemässen Blockcopolymere, Polymere oder Kontaktlinsen bereitzustellen, Verbindungen der Formel III, 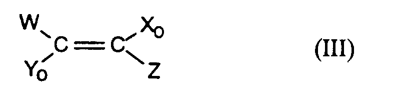 die, symbolisiert durch den Buchstaben A, in das Blockcopolymere der Formel I in Gestalt der Teilformel IV eingebaut werden, 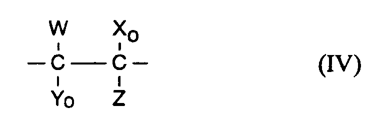 wobei die Substituenten W, Xₒ, Yₒ und Z die folgenden Bedeutungen aufweisen: drei dieser Substituenten bedeuten Wasserstoff und der vierte Substituent ist ausgewählt unter Acyl, Halogen, einem heterocyclischen Rest oder Aryl, oder zwei dieser Substituenten bedeuten Wasserstoff, der dritte bedeutet Niederalkyl, und der vierte Substituent ist ausgewählt unter Acyl, Halogen, einem heterocyclischen Rest oder Aryl, oder zwei dieser Substituenten bedeuten Wasserstoff und die beiden anderen Substituenten bilden gemeinsam eine Kohlenwasserstoffbrücke, die ununterbrochen oder durch ein oder zwei Heteroatome unterbrochen ist, oder die beiden anderen Substituenten bedeuten unabhängig voneinander Acyl. Die Monomere der Formel III sind entweder hydrophile Vinylmonomere oder hydrophobe Vinylmonomere.

Aryl bedeutet insbesondere einen aromatischen Kohlenwasserstoffrest mit 6 bis 15 Kohlenstoffatomen, wie Phenyl oder Phenyl, das durch einen oder mehrere, insbesondere bis zu drei, Reste der Art Niederalkyl, Niederalkoxy, Halogen, Amino oder Hydroxy substituiert ist. Beispiele sind Phenyl oder Tolyl.

Halogen bedeutet insbesondere Chlor, Brom oder Fluor, kann jedoch auch für Iod stehen.

Ein heterocyclischer Rest ist insbesondere ein 5- oder 6-gliedriger aromatischer oder gesättigter Ring mit ein oder zwei Heteroatomen, wie Sauerstoff- oder Stickstoffatomen, insbesondere mit ein oder zwei Stickstoffatomen. Hiervon sind auch Lactame erfasst.

Eine Kohlenwasserstoffbrücke, die ununterbrochen oder durch ein oder zwei Heteroatome unterbrochen ist, bedeutet insbesondere Niederalkylen oder durch Sauerstoff oder Stickstoff unterbrochenes Niederalkylen. Durch Stickstoff unterbrochenes Niederalkylen kann auch substituiert sein, z.B. durch Niederalkyl. Beispiele sind 1,3-Propylen, 2-Aza-1,3-Propylen oder N-Methyl-2-Aza-1,3-Propylen.

Acyl steht für Carboxy, Aroyl, Cycloalkanoyl oder Alkanoyl und insbesondere für Carboxy, unsubstituiertes oder substituiertes Aryloxycarbonyl, unsubstituiertes oder substituiertes Cycloalkyloxycarbonyl oder unsubstituiertes oder substituiertes Alkoxycarbonyl.

Aroyl bedeutet beispielsweise Benzoyl oder durch einen oder mehrere, insbesondere bis zu drei, Reste der Art Niederalkyl, Niederalkoxy, Halogen oder Hydroxy substituiertes Benzoyl, kann aber auch Phenylsulfonyl oder Phenyloxysulfonyl sowie durch Niederalkyl, Niederalkoxy, Halogen oder Hydroxy substituiertes Phenylsulfonyl oder Phenyloxysulfonyl bedeuten.

Alkanoyl bedeutet vorzugsweise Niederalkanoyl und ist z.B. Acetyl, Propanoyl oder Butanoyl.

Cycloalkanoyl bedeutet vorzugweise Cycloalkyloxycarbonyl mit bis zu 8 Kohlenstoffatomen und bedeutet z.B. Cyclohexyloxycarbonyl.

Unsubstituiertes Alkoxycarbonyl ist vorzugsweise Niederalkoxycarbonyl und bedeutet z.B. Methoxycarbonyl, Ethoxycarbonyl, Propyloxycarbonyl, Butoxycarbonyl, tert.-Butoxycarbonyl, tert-Butylmethyloxycarbonyl oder 2-Ethylhexyloxycarbonyl.

Unsubstituiertes Aryloxycarbonyl ist vorzugsweise Phenyloxycarbonyl.

Substituiertes Aryloxycarbonyl ist vorzugsweise durch einen oder mehrere, insbesondere bis zu drei, Reste der Art Niederalkyl, Niederalkoxy, Halogen oder Hydroxy substituiertes Phenyloxycarbonyl.

Substituiertes Alkoxycarbonyl ist vorzugsweise durch hydrophobe Gruppen, wie Halogen, z.B. Fluor, Siloxangruppen oder hydrophile Gruppen, wie Hydroxy, Amino, Mono- oder Diniederalkylamino, Isocyanato oder durch ein Niederalkylenglycol substituiert. Weitere Bedeutungen von substituiertem Alkoxycarbonyl, wie auch von substituiertem Aryloxycarbonyl und substituiertem Cycloalkyloxycarbonyl, werden implizit durch die nachfolgende Beschreibung von speziell geeigneten Vinylmonomeren der Formel III gegeben.

Die erfindungsgemäss verwendbaren hydrophilen Vinylmonomere sind vorzugsweise Acrylate und Methacrylate der Formel III, worin W und Yₒ für Wasserstoff stehen, Xₒ für Wasserstoff oder Methyl steht und Z eine Gruppe -Z¹-Z² bedeutet, worin Z¹ für -COO-steht, das über Sauerstoff an Z² gebunden ist, und Z² einen durch eine wasserlöslich machende Gruppe wie Carboxy, Hydroxy oder tert.-Amino, z.B. tert. Niederalkylamino mit 1 bis 7 Kohlenstoffatomen je Niederalkylgruppe, eine Polyethylenoxidgruppe mit 2-100 sich wiederholenden Einheiten, bevorzugt mit 2-40 sich wiederholenden Einheiten, oder eine Sulfat-, Phosphat-, Sulfonat- oder Phosphonatgruppe einfach oder mehrfach substituierten Kohlenwasserstoffrest mit 1 bis 10 Kohlenstoffatomen bedeutet, wie z.B. einen entsprechend substituierten Alkyl-, Cycloalkyl- oder Phenylrest oder eine Kombination solcher Reste, wie Phenylalkyl oder Alkylcycloalkyl;
ferner Acrylamide und Methacrylamide der Formel III, worin W und Yₒ für Wasserstoff stehen, Xₒ für Wasserstoff oder Methyl steht und Z Aminocarbonyl oder Diniederalkylaminocarbonyl bedeutet;
Acrylamide und Methacrylamide der Formel III, worin W und Yₒ für Wasserstoff stehen, Xₒ für Wasserstoff oder Methyl steht und Z monosubstituiertes Aminocarbonyl bedeutet, das durch eine wie vorstehend definierte Gruppe Z² oder Niederalkyl substituiert ist;
Maleinate und Fumarate der Formel III, worin W und Xₒ (oder W und Z) für Wasserstoff stehen, und Yₒ und Z (oder Xₒ und Yₒ) unabhängig voneinander eine Gruppe -Z¹-Z² bedeuten, worin Z¹ und Z² wie vorstehend definiert sind;
Crotonate der Formel III, worin W und Xₒ für Wasserstoff stehen, Yₒ für Methyl steht und Z eine Gruppe -Z¹-Z² bedeutet, worin Z¹ und Z² wie vorstehend definiert sind;
Vinylether der Formel III, worin W, Xₒ und Yₒ für Wasserstoff stehen und Z eine Gruppe -Z¹-Z² bedeutet, worin Z¹ für Sauerstoff steht und Z² wie vorstehend definiert ist;
Vinyl-substituierte fünf- oder sechsgliedrige Heterocyclen mit ein oder zwei Stickstoffatomen sowie N-Vinyllactame, wie N-Vinyl-2-pyrrolidon, der Formel III, worin W, Xₒ und Yₒ für Wasserstoff stehen und Z einen fünf- oder sechsgliedrigen heterocyclischen Rest mit ein oder zwei Stickstoffatomen bedeutet, sowie den über Stickstoff gebundenen Rest eines Lactams, z.B. denjenigen von 2-Pyrrolidon;
und vinylisch ungesättigte Carbonsäuren der Formel III mit insgesamt 3 bis 10 Kohlenstoffatomen, wie Methacrylsäure, Crotonsäure, Fumarsäure oder Zimtsäure.

Bevorzugt sind z.B. durch Hydroxy substituierte C₂-C₄-Alkyl(meth)acrylate, fünf- bis siebengliedrige N-Vinyllactame, N,N-Di-C₁-C₄-alkyl(meth)acrylamide und vinylisch ungesättigte Carbonsäuren mit insgesamt 3 bis 5 Kohlenstoffatomen.

Zu den verwendbaren wasserlöslichen Monomeren gehören: 2-Hydroxyethyl-, 2- und 3-Hydroxypropyl-, 2,3-Dihydroxypropyl-, Polyethoxyethyl- und Polyethoxypropylacrylate und -methacrylate sowie die entsprechenden Acrylamide und Methacrylamide, Acrylamid und Methacrylamid, N-Methylacrylamid und -methacrylamid, Bisaceton-acrylamid, 2-Hydroxyethylacrylamid, Dimethylacrylamid und -methacrylamid sowie Methylolacrylamid und -methacrylamid, N,N-Dimethyl- und N,N-Diethylaminoethylacrylat und -methacrylat sowie die entsprechenden Acrylamide und Methacrylamide, N-tert.-Butylaminoethylmethacrylat und -methacrylamid, 2- und 4-Vinylpyridin, 4- und 2-Methyl-5-vinylpyridin, N-Methyl4-vinylpiperidin, 1-Vinyl- und 2-Methyl-1-vinyl-imidazol, Dimethylallylamin und Methyldiallylamin sowie para-, meta- und ortho-Aminostyrol, Dimethylaminoethylvinylether, N-Vinylpyrrolidon und 2-Pyrrolidinoethylmethacrylat, Acryl- und Methacrylsäure, Itaconsäure, Zimtsäure, Crotonsäure, Fumarsäure, Maleinsäure und deren Hydroxyniederalkylmono- und -diester, wie 2-Hydroxyethyl- und Di-(2-hydroxy)ethylfumarat, -maleinat und -itaconat, sowie 3-Hydroxypropyl-butylfumarat und Di-polyalkoxyalkyl-fumarate, -maleinate und -itaconate, Maleinsäureanhydrid, N-Methyl-maleinsäureimid, Natriumacrylat und -methacrylat, 2-Methacryloyloxyethylsulfonsäure, 2-Acrylamido-2-methylpropansulfonsäure, 2-Phosphatoethylmethacrylat, Vinylsulfonsäure, Phenylvinylsulfonat, Natriumvinylsulfonat, p-Styrolsulfonsäure, Natrium-p-styrolsulfonat und Allylsulfonsäure, N-Vinylpyrrolidon, N-Vinylpyridon, N-Vinylcaprolactam, ferner die quaternisierten Derivate kationischer Monomerer, welche man durch Quaternierung mit ausgewählten Alkylierungsmitteln, z.B. halogenierten Kohlenwasserstoffen wie Methyliodid, Benzylchlorid oder Hexadecylchlorid, Epoxiden wie Glycidol, Epichlorhydrin oder Ethylenoxid, Acrylsäure, Dimethylsulfat, Methylsulfat und Propansulton erhält.

Eine vollständigere Liste im Zusammenhang mit dieser Erfindung verwendbarer, wasserlöslicher Monomerer findet sich in: R.H. Yocum und E.B. Nyquist, Functional Monomers [Funktionelle Monomere], Band 1, S. 424-440 (M. Dekker, N.Y. 1973).

Bevorzugte hydrophile Vinylmonomere sind 2-Hydroxyethylmethacrylat, 3-Hydroxypropylmethacrylat, N-Vinyl-2-pyrrolidon, Polyethylenglykolmethacrylat, insbesondere mit einem Ethylenglykolanteil eines Molekulargewichts von etwa 400, N,N-Dimethylacrylamid sowie Acryl- und Methacrylsäure.

Als hydrophobe Vinylmonomere, die gegebenenfalls erfindungsgemäss verwendet werden, kommen beispielsweise in Betracht:

Acrylate und Methacrylate der Formel III, worin worin W und Yₒ für Wasserstoff stehen, Xₒ für Wasserstoff oder Methyl steht und Z eine Gruppe -Z¹-Z³ bedeutet, worin Z¹ für -COO- steht, das über Sauerstoff an Z³ gebunden ist, und Z³ eine lineare oder verzweigte aliphatische, eine cycloaliphatische oder eine aromatische Gruppe mit 1 bis 21 Kohlenstoffatomen ist, wie z.B. ein entsprechend substituierter Alkyl-, Cycloalkyl- oder Phenylrest oder eine Kombination solcher Reste, wie Phenylalkyl oder Alkylcycloalkyl, die Ether- oder Thioetherbindungen, Sulfoxid oder Sulfongruppen oder eine Carbonylgruppe enthalten kann; oder Z³ eine heterocyclische Gruppe ist, die Sauerstoff-, Schwefel- oder Stickstoffatome und 5 oder 6, oder falls sie bicyclisch ist, bis zu 10 Ringatome enthält, oder eine Polypropylenoxid- oder Poly-n-butylenoxid-Gruppe mit 2 bis 50 wiederkehrenden Alkoxyeinheiten, oder Z³ eine Alkylgruppe mit 1-12 Kohlenstoffatomen ist, die Halogenatome enthält, insbesondere Fluoratome, oder Z³ eine Siloxangruppe mit 1 bis 6 Si-Atomen ist;
Acrylamide und Methacrylamide der Formel III, worin W und Yₒ für Wasserstoff stehen, Xₒ für Wasserstoff oder Methyl steht und Z monosubstituiertes Aminocarbonyl bedeutet, das durch eine wie vorstehend definierte Gruppe Z³ substituiert ist;
Maleinate und Fumarate der Formel III, worin W und Xₒ (oder W und Z) für Wasserstoff stehen, und Yₒ und Z (oder Xₒ und Yₒ) unabhängig voneinander eine Gruppe -Z¹-Z³ bedeuten, worin Z¹ und Z³ wie vorstehend definiert sind;
Itaconate der Formel III, worin W und Yₒ für Wasserstoff stehen, Xₒ eine Gruppe -Z¹-Z³ bedeutet, worin Z¹ und Z³ wie vorstehend definiert sind und Z eine Gruppe -CH₂-Z¹-Z³ bedeutet, worin Z¹ und Z³ wie vorstehend definiert sind;
Crotonate der Formel III, worin W und Xₒ für Wasserstoff stehen, Yₒ für Methyl steht und Z eine Gruppe -Z¹-Z³ bedeutet, worin Z¹ und Z³ wie vorstehend definiert sind;
Vinylester der Formel III, worin W, Yₒ, und Xₒ für Wasserstoff stehen und Z eine Gruppe -Z¹-Z³ bedeutet, worin Z¹ für -COO- steht, das über Kohlenstoff an Z³ gebunden ist, und Z³ wie vorstehend definiert ist;
Vinylether der Formel III, worin W, Xₒ und Yₒ für Wasserstoff stehen und Z eine Gruppe -Z¹-Z³ bedeutet, worin Z¹ für Sauerstoff steht und Z³ wie vorstehend definiert ist;

Bevorzugt sind insbesondere C₁-C₄-Alkylester oder C₅-C₇-Cycloalkylester von vinylisch ungesättigten Carbonsäuren mit 3 bis 5 Kohlenstoffatomen.

Beispiele geeigneter hydrophober Monomerer sind: Methyl-, Ethyl-, Propyl-, Isopropyl-, Butyl-, Isobutyl-, tert-Butyl-, Ethoxyethyl-, Methoxyethyl-, Benzyl-, Phenyl-, Cyclohexyl-, Trimethylcyclohexyl-, Isobornyl-, Dicyclopentadienyl-, Norbornylmethyl-, Cyclododecyl-, 1,1,3,3-Tetramethylbutyl-, n-Butyl-, n-Octyl-, 2-Ethylhexyl-, Decyl-, Dodecyl-, Tridecyl-, Octadecyl-, Glycidyl-, Ethylthioethyl-, Furfuryl-, Tri-, Tetra- und Pentasiloxanylpropyl-acrylate und -methacrylate, sowie die entsprechenden Amide; N-(1,1-Dimethyl-3-oxobutyl)-acrylamid; Mono- und Dimethyl-fumarat, -maleat und -itaconat; Diethylfumarat; Isopropyl- und Diisopropyl-fumarat und -itaconat; Mono- und Diphenyl- und Methylphenyl-fumarat und -itaconat; Methyl- und Ethylcrotonat; Methylvinylether und Methoxyethylvinylether; Vinylacetat, Vinylpropionat, Vinylbenzoat, Acrylnitril, Vinylidenchlorid, Styrol, α-Methylstyrol und tert-Butylstyrol.

Bevorzugte hydrophobe Vinylmonomere sind Methylmethacrylat, n-Butylmethacrylat, Isopropylmethacrylat, Isobutylmethacrylat, Cyclohexylmethacrylat oder ein Gemisch davon.

Unter den vorstehend genannten Vinylmonomeren sind zwei spezielle Typen von hydrophoben Vinylmonomeren im erfindungsgemässen Zusammenhang besonders erwähnenswert, nämlich Siloxanmonovinylkomponenten und fluorhaltige Vinylverbindungen.

Besonders bevorzugte Siloxanmonovinylkomponenten sind Verbindungen der Formel III, worin W und Yₒ für Wasserstoff stehen, Xₒ für Wasserstoff oder Methyl steht und Z eine Gruppe -Z¹-Z⁴ bedeutet, worin Z¹ für -COO- steht, das über Sauerstoff an Z⁴ gebunden ist und worin Z⁴ ein oder mehrfach, z.B. drei- bis neunfach, durch Triniederalkylsilyloxy substituiertes Silyl-niederalkyl ist. Unter Silyl-niederalkyl wird in diesem Zusammenhang ein durch ein oder mehrere Siliciumatome substituierter Niederalkylrest verstanden, dessen freie Valenzen an den Siliciumatomen insbesondere durch Triniederalkylsilyloxy abgesättigt sind. Speziell hervorzuhebende Einzelverbindungen sind beispielsweise Tris(trimethylsiloxy)silylpropylmethacrylat und Tris(tris(trimethylsiloxy)siloxy)silylpropylmethacrylat.

Besonders bevorzugte fluorhaltige Vinylverbindungen sind Verbindungen der Formel III, worin W und Yₒ für Wasserstoff stehen, Xₒ für Wasserstoff oder Methyl steht und Z eine Gruppe -Z¹-Z⁵ bedeutet, worin Z¹ für -COO- steht, das über Sauerstoff an Z⁵ gebunden ist und worin Z⁵ durch Fluor substituiertes Alkyl, insbesondere Niederalkyl ist. Spezielle Beispiele hierfür sind 2,2,2-Trifluorethylmethacrylat, 2,2,3,3-Tetrafluorpropylmethacrylat, 2,2,3,3,4,4,5,5-Octafluorpentylmethacrylat und Hexafluorisopropylmethacrylat.

Wie bereits erwähnt sind Copolymere der Formel I besonders bevorzugt, bei denen- es sich um Triblockcopolymere, Kammpolymere oder Sternpolymere handelt. Für alle diese drei Typen von Copolymeren der Formel I, insbesondere aber für Triblockcopolymere, gilt, dass solche besonders bevorzugt sind, worin Macro für den Rest eines Polysiloxans oder eines fluorierten Polyethers steht und der Teil A von einem hydrophilen Vinylmonomer abgeleitet ist, das eine reaktive Gruppe aufweist. Bei der reaktiven Gruppe handelt es sich insbesondere um Hydroxy oder Isocyanato. Beispiele für Vinylmonomere, die derartige Gruppen aufweisen, sind Hydroxy-niederalkyl(meth)acrylate oder ein Isocyanato-niederalkyl(meth)acrylat, wie insbesondere Hydroxyethylmethacrylat oder Isocyanatoethylmethacrylat.

Ferner bevorzugt sind Copolymere der Formel I, insbesondere Triblockcopolymere, worin Macro für den Rest eines Polysiloxans oder eines fluorierten Polyethers steht und der Teil A von einem hydrophilen Vinylmonomer abgeleitet ist, das keine reaktive Gruppe aufweist. Ein derartiges Vinylmonomer ist insbesondere ein Vinyllactam, vor allem N-Vinylpyrrolidon. Ferner sind auch Copolymere der Formel I, insbesondere Triblockcopolymere, bevorzugt, worin Macro für den Rest eines hydrophilen Macromeren steht, wie vorstehend definiert, und der Teil A von einem hydrophoben Vinylmonomer abgeleitet ist.

Wie bereits erwähnt, werden die erfindungsgemässen Polymere vorzugweise ausgehend von einer Verbindung der Formel C und einem Vinylmonomer in Gegenwart von einem Vernetzer hergestellt.

Geeignete Vernetzer sind insbesondere oligoolefinische, insbesondere diolefinische Monomere, z.B. Allylacrylat und -methacrylat, Ethylenglykol-, Diethylenglykol-, Triethylenglykol-, Tetraethylenglykol- und allgemein Polyethylenoxidglykoldiacrylate und -dimethacrylate 1,4-Butandiol- und Polyn-butylenoxidglykoldiacrylate und -dimethacrylate, Propylenglykol- und Polypropylenoxidglykoldiacrylate und -dimethacrylate, Thiodiethylenglykoldiacrylat und -dimethacrylat, Di-(2-hydroxyethyl)sulfondiacrylat und -dimethacrylat, Neopentylglykoldiacrylat und -dimethacrylat, Trimethylolpropan-tri- und -tetraacrylat, Pentaerythrit-tri- und -tetraacrylat, Divinylbenzol, Divinylether, Divinylsulfon, Disiloxanyl-bis-3-hydroxypropyldiacrylat oder-methacrylat und verwandte Verbindungen. Ethylenglykoldimethacrylat ist bevorzugt.

Als Vernetzer kommen auch Oligovinylmacromere, z.B. Divinylmacromere in Frage, wie sie z.B. in der US-A4,136,250 beschrieben sind. Ferner sind als Vernetzer im erfindungsgemässen Zusammenhang auch Oligovinylsiloxanverbindungen geeignet, z.B. Bis(meth)acryloxy-niederalkyl-siloxane mit bis zu 10 Siliciumatomen. Beispiele hierfür sind 3,5-Bis(3-methacroyloxypropyl)-3,5-bis(trimethylsiloxy)-1,1,1,7,7,7-hexamethyltetrasiloxan und 1,3-Dimethacryloxypropyl-tetramethyldisiloxan.

Die bei der Herstellung der erfindungsgemässen Copolymere, Polymere und Pfropfcopolymere zur Anwendung kommenden Ausgangsmaterialien, z.B. solche der Formeln A, B, III und die Vernetzer sind an sich bekannt und / oder hierin beschrieben.

Die Verbindungen der Formeln II können in an sich bekannter Weise durch die Umsetzung von Diisocyanaten mit den entsprechenden H-aciden Photoinitiatoren hergestellt werden. Die Verbindungen werden in hohen Ausbeuten und Reinheiten erhalten, selbst wenn im Photoinitiator gleichzeitig zwei verschieden reaktive H-acide Gruppen zugegen sind, zum Beispiel zwei OH-Gruppen. Besonders vorteilhaft ist es, Diisocyanate mit Isocyanatgruppen unterschiedlicher Reaktivität zu verwenden, weil hiermit die Bildung von Isomeren und Diaddukten weitgehend unterdrückt Werden kann. Die unterschiedliche Reaktivität kann zum Beispiel wie zuvor beschrieben durch eine sterischen Hinderung bewirkt werden. Die unterschiedliche Reaktivität kann auch durch eine Verkappung einer Isocyanatgruppe im Diisocyanat erzielt werden, zum Beispiel mit Carbonsäuren oder Hydroxylamin. Die Verbindungen der Formel IIa sind aus der EP-A-632329 bekannt.

Verbindungen der Formel (IIb) lassen sich dadurch herstellen, dass man eine Verbindung der Formel IIc 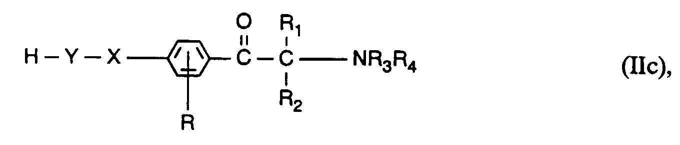 worin X, Y, R, R₁, R₂, R₃ und R₄ die zuvor angegebenen Bedeutungen haben, vorzugsweise in einem inerten organischen Lösungsmittel mit einem Diisocyanat der Formel IId oder einem solchen gegebenenfalls monoverkappten Diisocyanat,

OCN-R₅-NCO (IId),

worin R₅ die zuvor angegebenen Bedeutungen hat, umsetzt.

Verkappungsmittel sind aus der Urethanchemie bekannt. Es kann sich zum Beispiel um Phenole (Kresol, Xylenol), Lactame (ε-Caprolactam), Oxime (Acetoxim, Benzophenonoxim), H-aktive Methylenverbindungen (Diethylmalonat, Ethylacetoacetat), Pyrazole oder Benztriazole handeln. Verkappungsmittel sind zum Beispiel von Z. W. Wicks, Jr. in Progress in Organic Coatings, 9 (1981), Seiten 3-28 beschrieben.

Die Edukte vom Typ der Formel IIc sind bekannt und werden z.B. in EP-A-284,561, EP-A-117,233 oder EP-A-088,050 beschrieben.

Geeignete inerte Lösungsmittel sind aprotische unpolare oder polare Lösungsmittel wie zum Beispiel Kohlenwasserstoffe (Petrolether, Methylcyclohexan, Benzol, Toluol, Xylol), Halogenkohlenwasserstoffe (Chloroform, Methylenchlorid, Trichlorethan, Tetrachlorethan, Chlorbenzol), Ether (Diethylether, Dibutylether, Ethylenglykoldimethylether, Diethylenglykoldimethylether, Tetrahydrofuran (THF), Dioxan), Ketone (Aceton, Dibutylketon, Methyl-isobutylketon), Carbonsäureester und Lactone (Essigsäureethylester, Butyrolacton, Valerolacton), alkylierte Carbonsäureamide (N,N-Dimethylacetamid (DMA) oder N,N-Dimethylformamid (DMF), N-Methyl-2-pyrrolidon (NMP)), Nitrile (Acetonitril), Sulfone und Sulfoxide (Dimethylsulfoxid (DMSO), Tetramethylensulfon). Bevorzugt werden polare Lösungsmittel verwendet.

Die Reaktanden werden vorteilhaft in äquimolaren Mengen eingesetzt. Die Reaktionstemperatur kann zum Beispiel von 0 bis 200°C betragen. Bei der Verwendung von Katalysatoren können die Temperaturen zweckmässig im Bereich von -20° bis 60°C und vorzugsweise im Bereich von -10° bis 50°C liegen. Geeignete Katalysatoren sind zum Beispiel Metallsalze wie Alkalimetallsalze von Carbonsäuren, tertiäre Amine, zum Beispiel (C₁-C₆-Alkyl)₃N (Triethylamin, Tri-n-butylamin), N-Methylpyrrolidin, N-Methylmorpholin, N,N-Dimethylpiperidin, Pyridin und 1,4-Diaza-bicyclooctan. Als besonders effektiv haben sich Zinnverbindungen erwiesen, besonders Alkylzinnsalze von Carbonsäuren, wie zum Beispiel Dibutylzinndilaurat, oder z.B. Zinndioctoat.

Sofern in Verbindungen der Formel IIc freie NH-Gruppen vorhanden sind, so können diese während der Reaktion mit einem Diisocyanat mit geeigneten Schutzgruppen zuerst geschützt und nachher durch Abspaltung der Schutzgruppen wieder freigesetzt werden. Geeignete Schutzgruppen sind dem Fachmann bekannt. Repräsentative Beispiele können beispielsweise aus T.W. Greene, "Protective Groups in Organic Synthesis", Wiley Interscience, 1981, entnommen werden.

Die Isolierung und Reinigung der hergestellten Verbindungen erfolgt nach bekannten Verfahren wie zum Beispiel Extraktion, Kristallisation, Umkristallisation oder chromatographischen Reinigungsmethoden. Die Verbindungen werden in hohen Ausbeuten und Reinheiten erhalten. Die Ausbeuten bei nicht optimierten Verfahren können über 85 % der Theorie betragen.

Die Umsetzung eines Makromeren der Formel A mit einem Photoinitiator der Formel B kann einfach und auf in der Urethanchemie an sich bekannte Weise erfolgen.

Die Umsetzung eines Reaktionsproduktes, gebildet aus einem Makromer der Formel A und einem Photoinitiator der Formel B, mit einem Vinylmonomer, das als Bestandteil "A" in das Copolymer eingebaut wird, kann ebenfalls auf an sich bekannte Weise erfolgen. So kann ein Reaktionsprodukt, gebildet aus einem Makromer der Formel A und einem Photoinitiator der Formel B, mit einem Vinylmonomer, das als Bestandteil "A" in das Copolymer eingebaut wird, bei Raumtemperatur oder bei einer Temperatur bis maximal zur Siedetemperatur des gegebenenfalls verwendeten Lösungsmittels in Abwesenheit oder Gegenwart eines geeigneten Lösungsmittels copolymerisiert werden. Ein geeignetes Lösungsmittel ist beispielsweise ein Kohlenwasserstoff, wie Hexan, Benzol oder Toluol, oder ein Ether, wie Diethylether oder Tetrahydrofuran, oder ein Alkohol, wie Ethanol oder Isopropanol, oder ein Amid, wie N-Methylpyrrolidon, oder Dimethylsulfoxid, oder ein Gemisch von mehreren dieser Lösungsmittel. Die Reinigung erfolgt auf an sich bekannte Weise. Für die Vernetzungsreaktion zu erfindungsgemässen Polymeren oder Pfropfpolymeren können grundsätzlich gleiche Bedingungen angewandt werden.

Geeignete Olefine der genannten Pfropfpolymerisation sind zum Beispiel Acrylamid, N,N-Dimethylacrylamid, Methacrylamid, Hydroxyethylmethacrylat, Glycerylmethacrylat, Oligoethylenoxidmono- und -bisacrylate, Ethylenglykoldimethacrylat, Methylenbisacrylamid, Vinylcaprolactam, Acrylsäure, Methacrylsäure, Fumarsäuremonovinylester, Vinyltrifluoracetat und Vinylencarbonat, wobei reaktive Ester anschliessend gegebenenfalls hydrolysiert werden können.

In bestimmten Fällen kann es von Vorteil sein, Gemische von zwei oder mehr Photoinitiatoren zu verwenden. Selbstverständlich können auch Gemische mit bekannten Photoinitiatoren verwendet werden, z.B. Gemische mit Benzophenon, Acetophenonderivaten, Benzoinethern oder Benzilketalen.

Zur Beschleunigung der Photopolymerisation können Amine zugesetzt werden, wie z.B. Triethanolamin, N-Methyl-diethanolamin, p-Dimethylaminobenzoesäure-ethylester oder Michlers Keton. Die Wirkung der Amine kann verstärkt werden durch den Zusatz von aromatischen Ketonen vom Typ des Benzophenons.

Eine Beschleunigung der Photopolymerisation kann weiterhin durch Zusatz von Photosensibilisatoren geschehen, welche die spektrale Empfindlichkeit verschieben bzw. verbreitern. Dies sind insbesondere aromatische Carbonylverbindungen wie z.B. Benzophenon-, Thioxanthon-, Anthrachinon- und 3-Acylcumarinderivate sowie 3-(Aroylmethylen)-thiazoline.

Die Wirksamkeit der Photoinitiatoren lässt sich steigern durch Zusatz von Titanocenderivaten mit fluororganischen Resten, wie sie in den EP-A-122,223 und EP-A-186,626 beschrieben sind, z.B. in einer Menge von 1-20 %. Beispiele für solche Titanocene sind Bis(methylcyclopentadienyl)bis(2,3,6-trifluorphenyl)-titan, Bis(cyclopentadienyl)bis-(4-dibutylamino-2,3,5,6-tetrafluorphenyl)-titan, Bis(methylcyclopentadienyl)-2-(trifluormethyl)phenyl-titan-isocyanat, Bis(cyclopentadienyl)-2-(trifluormethyl)phenyltitan-trifluoracetat oder Bis(methylcyclopentadienyl)bis(4-decyloxy-2,3,5,6-tetrafluorphenyl)-titan. Für diese Gemische eignen sich vor allem flüssige α-Aminoketone.

Aus den erfindungsgemässen segmentierten Copolymeren und insbesondere aus den erfindungsgemässen Polymeren können auf an sich bekannte Weise Formkörper, insbesondere Kontaktlinsen hergestellt werden. Dazu werden z.B. die erfindungsgemässen Polymere in zylindrischer Form polymerisiert, und die erhältlichen Stäbe nach Entformung in Scheiben oder Knöpfe zerteilt, die weiter mechanisch bearbeitet werden können, insbesondere durch Drehverfahren. Darüberhinaus können die erfindungsgemässen Formkörper respektive Linsen auch nach anderen an sich bekannten Verfahren wie Giessen in statischen Formen, Rotationsgiessen, Verpressen, Tiefziehen, Warmformen, Drehen oder Laserbearbeitung hergestellt werden. Diese Verfahrensschritte sind an sich bekannt und bedürfen daher für den Fachmann keiner detaillierten Erläuterung.

Die Herstellung erfolgt vorzugsweise unter einer inerten Atmosphäre, wenn sie in offenen Formen durchgeführt wird. Bekanntlich hemmt Sauerstoff die Polymerisation und führt zu verlängerten Polymerisationszeiten. Werden geschlossene Formen zur Bildung des Polymerisats verwendet, so bestehen die Formen vorteilhafterweise aus inerten Materialien mit niedriger Sauerstoffdurchlässigkeit und mit nicht-klebenden Eigenschaften. Beispiele für geeignete Formmaterialien sind Polytetrafluorethylen, wie Teflon@, Silikonkautschuk, Polyethylen, Polypropylen und Polyester wie Mylar® Bei Einsatz eines geeigneten Entformungsmittels sind auch Formen aus Glas und Metall verwendbar.

Giessen in statischen Formen kann beispielsweise, wenn Formen mit Innenkurve und Aussenkurve verwendet werden, unmittelbar zu Kontaktlinsen führen. So können Kontaktlinsen durch Polymerisation in geeigneten Formen direkt ("full mold"-Verfahren) oder mit nur einer fertigen Fläche ("semi mold"-Verfahren) hergestellt werden.

Rotationsgiessen (spin casting) lässt sich erfindungsgemäss ebenfalls anwenden, indem eine Lösung der erfindungsgemässen Ausgangsmaterialien in eine Form für Rotationsguss eingebracht wird, worauf die Form in Rotation versetzt wird. Dabei verdampft das Lösungsmittel. Die fertige Kontaktlinse, deren Abmessungen sich durch die Abmessungen der Form, die Rotationsgeschwindigkeit und die Viskosität der eingebrachten Lösung steuern lassen, bleibt in der Form zurück.

Verpressen geschieht erfindungsgemäss z.B. durch Formpressen einer Folie aus dem erfindungsgemässen Polymer. Eine Folie aus dem Polymer kann auf an sich bekannte Weise beispielsweise durch Giessen einer Lösung hergestellt werden.

Aus einer z.B. wie vorstehend erwähnt hergestellten Folie kann eine Kontaktlinse auch auf an sich bekannte Weise durch Tiefziehen oder Warmformen hergestellt werden.

Drehen bietet sich als letzter Verfahrensschritt zur Herstellung von erfindungsgemässen Kontaktlinsen ebenfalls an. Dies gilt immer dann, wenn ein z.B. nach einem der vorstehend genannten Verfahren erhältlicher Rohling noch weiterer Bearbeitung bedarf. Unter Drehen wird das an sich bekannte spanabhebende Bearbeitungsverfahren von Kontaktlinsen-Rohlingen verstanden. Entsprechende Rohlinge lassen sich z.B. durch Extrusion von Rundstäben und deren Zerteilen oder Giessen aus einer Lösung herstellen. Unter den Begriff Kontaktlinsen-Rohling fallen in diesem Zusammenhang Knöpfe (buttons) oder semi-mold-Produkte, wie z.B. Innenkurvenrohlinge. Typische Rohlinge weisen Dicken von 4 oder 6 mm und Durchmesser von 10 bis 17, z.B. 12 oder 14 mm auf. Für weiche Materialien kann es erforderlich sein, diese vor einer entsprechenden Bearbeitung zu gefrieren, insbesondere unter den Erweichungspunkt, und die hierfür erforderlichen Temperaturen nötigenfalls während der Bearbeitung aufrecht zu erhalten.

Auch die Laserbearbeitung lässt sich erfindungsgemäss anwenden, wobei man von Rohlingen oder nach einem der anderen Verfahren hergestellten Kontaktlinsen ausgeht, sofern letztere noch einer zusätzlichen Feinbearbeitung ihrer Oberfläche bedürfen.

Die nachfolgenden Beispiele erläutern den Gegenstand der Erfindung, ohne ihn jedoch, etwa auf den Umfang der Beispiele, zu beschränken. Prozente bei Mengenangaben sind Gewichtsprozente soweit nicht ausdrücklich anders angegeben. In den nachfolgenden Beispielen sind Temperaturen, wenn nicht anders angegeben, in Grad Celsius offenbart, Molekulargewichte, wie auch sonst in dieser Beschreibung, sind mittlere Molekulargewichte (Bezeichnung: "Mw"), wenn nicht ausdrücklich anders angegeben.

### A-Beispiele: Herstellung von Aza-photoinitiatoren

### Beispiel A1

2-Dimethylamino-2-benzyl-1-(4-(2-hydroxyethoxy)phenyl)-butan-1-on.

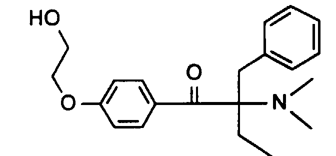

Die Herstellung der Titelverbindung erfolgt in Anlehnung an die in EP-A-284,561 beschriebene Synthese.

### Beispiel A2

2-Ethyl-2-dimethvlamino-1-(4-(2-hydroxyethoxy)phenyl)-pent-4-en-1-on.

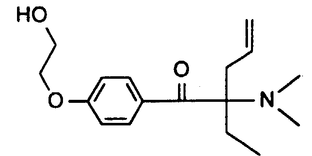

In Analogie zu Beispiel A1 wird die Titelverbindung in quantitaiver Ausbeute hergestellt. Es verbleiben gelbliche Kristalle vom Smp. 80 - 82°C.

### Beispiel A3

1-(4-(2-Hydroxyethylthio)phenyl)-2-methyl-2-morpholino-propan-1-on.

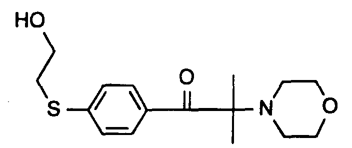

Die Herstellung der Titelverbindung ist in EP-A-088,050 beschrieben.

### Beispiel A4

1-(4-(2-Hydroxyethoxy)phenyl)-2-methyl-2-morpholino-provan-1-on.

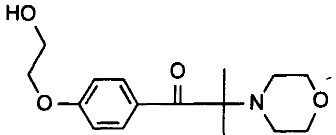

In Analogie zu Beipiel A3 wird die Titelverbindung hergestellt.

### Beispiel A5

Herstellung der nachstehenden Verbindung:

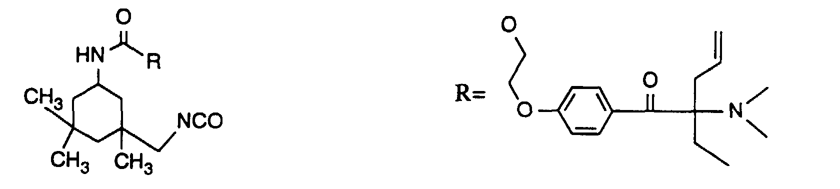

In einem 100 ml Kolben mit Rückflusskühler, Thermometer, Rührer und Stickstoffeinleitungsrohr werden 2.92 g (10 mmol) 2-Ethyl-2-dimethylamino-1-(4-(2-hydroxyethoxy)-phenyl)-pent-4-en-1-on (aus Beispiel A2) in 30 ml trockenem Methylenchlorid gelöst, und mit 2.22 g (10 mmol) IPDI gelöst in 30 ml trockenem Methylenchlorid vermischt. Hierzu gibt man 2.0 mg des Katalysators DBTDL und rührt 72 Std. bei RT. Der Reaktionsverlauf wird mit DC verfolgt (Laufmittel ist Toluol / Aceton 6:1). Danach wird die Reaktionslösung in Wasser eingerührt. Die organische Phase wird abgetrennt und noch zweimal mit Wasser gewaschen. Die organische Phase wird über MgSO₄ getrocknet und am RV eingeengt. Der verbleibende Rückstand wird säulenchromatographisch gereinigt (Toluol / Aceton 6:1). Es verbleiben 3.4 g (66%) eines gelben Oeles. Die Struktur wird mit Protonen-NMR, IR und Elementaranalyse verifiziert.

### Beispiel A6

In Analogie zu Beispiel A5 wird das nachfolgende Isocyanat hergestellt aus 1.17 g (4 mmol) 1 (4-(2-Hydroxyethoxy)phenyl)-2-methyl-2-morpholino-propan-1-on (aus Beispiel A4), 0.7 g (4 mmol) 2,4-TDI mit DBTDL als Katalysator in Methylenchlorid. Nach der Zugabe von 50 ml Ether und 200 ml Petrolether zum RG, fällt die Zielverbindung in kristalliner Form aus. Diese wird abfiltriert, mit Petrolether gewaschen und dann im Vakuum getrocknet. Man erhält die folgende Verbindung mit einem Smp. von 97 - 102°C:

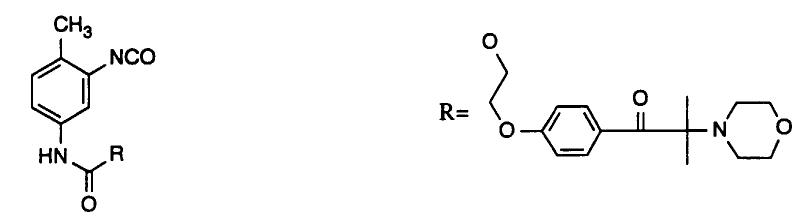

### Beispiele A7 und A8

In Analogie zu Beispiel A5 werden die nachstehenden Verbindungen hergestellt: 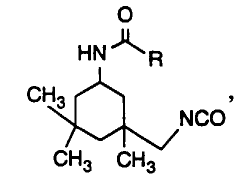 wobei R für die folgenden Reste steht:

Beispiel Nr. A7

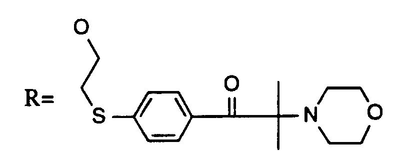

Beispiel Nr. A8

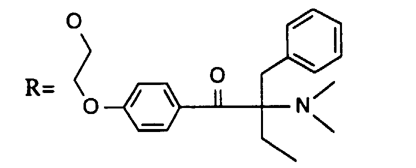

### B-Beispiele: Herstellung von Makrophotoinitiatoren:

### Beispiel B1

Herstellung eines oligomeren Photoinitiators: 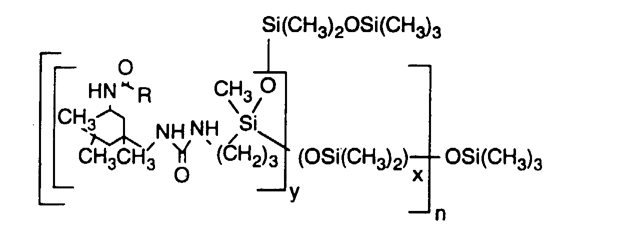 wobei 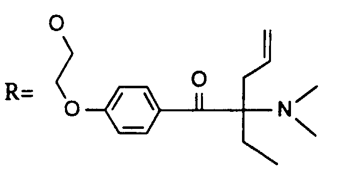 bedeutet, und x:y etwa 27:1 ist, und n 5 ist.

In einer Apparatur gemäss Beispiel A5 werden 0.7 g (1.3 mmol) des Isocyanates aus Beispiel A5, 20 ml trockenes Methylenchlorid und 2.55 g (0.51 mVal NH₂/g) Aminoalkylpolysiloxan KF 8003 (Shin Etsu, Japan) vorgelegt. Das Reaktionsgemisch wird 2 Stunden bei RT und 20 Minuten bei 40°C gerührt. Danach wird das Lösungsmittel am RV entfernt. Der Rückstand wird im Hochvakuum (40°C, 0.001 mbar(0.1 Pa)) von Lösungsmittelresten befreit. Man erhält die Titelverbindung in quantitativer Ausbeute. Im IR-Spektrum ist keine OCN-Bande vorhanden.

### Beispiel B2

In Analogie zu Beispiel B1 wird ein oligomerer Photoinitiator mit der Struktur gemäss Beispiel B1 hergestellt, ausgehend von 0.76 g (1.3 mmol) Isocyanat aus Beispiel A8 und 2.55 g (0.51 mVal NH₂/g) Aminoalkylpolysiloxan KF 8003 (Shin Etsu, Japan), wobei R die folgende Bedeutung hat:

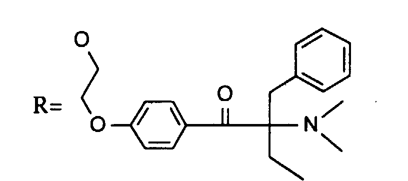

### Beispiel B3

In Analogie zu Beispiel B1 wird ein oligomerer Photoinitiator mit der folgenden Struktur hergestellt, ausgehend von 0.55 g (0.97 mmol) Isocyanat aus Beispiel A8 und 1.47 g (0.7 mVal NH₂/g) Aminoalkylpolysiloxan X-22-161B (Shin Etsu, Japan): 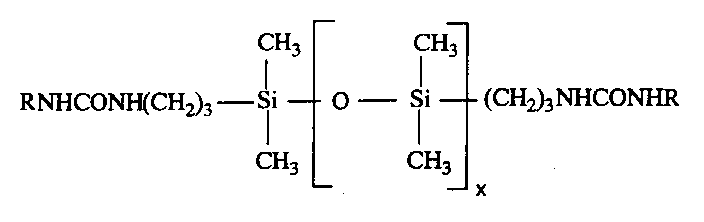 wobei x etwa 38 ist, und R dem um das Isocyanat verminderten Rest der Titelverbindung aus Beispiel A8 entspricht.

### Beispiel B4

In Analogie zu Beispiel B1 wird eine Lösung von 1.0 g (1.95 mmol) des Isocyanates aus Beispiel A5 in 20 ml trockenem Acetonitril mit 2.24 g (0.84 mVal NH₂ / g) Jeffamine ED 2001 (Texaco, USA) in 30 ml trockenem Acetonitril vermischt und 24 Stunden bei RT gerührt. Nach der Aufarbeitung erhält man 3.2 g (99 %) des nachstehenden Photoinitiators:

R-NHCONH-CHCH₃CH₂-(OCHCH₃CH₂)ₐ-(OCH₂CH₂)_{b}-(OCHCH₃CH₂)_{c}-NHCONH-R

wobei a + c = 2.5 und b = 40.5 bedeutet, und R dem um das Isocyanat verminderten Rest der Titelverbindung aus Beispiel A5 entspricht.

### Beispiel B5

In einer Apparatur gemäss Beispiel A5 werden unter Stickstoff 1.65 g Polyvinylalkohol (PVA) (Serva® 03/20, Molekulargewicht etwa 13 000) bei 80°C in trockenem NMP gelöst. Dann wird auf RT gekühlt und mit einer Lösung von 1.0 g (1.88 mmol) des Isocyanates aus Beispiel A7 in 10 ml trockenem NMP und mit 5 mg DBTDL als Katalysator versetzt. Dieses Gemisch wird dann für 48 Stunden auf 40°C erwärmt. Nach dieser Zeit ist mit IR kein OCN bei 2250 cm⁻¹ nachweisbar. Das RG wird auf RT gekühlt und mit 700 ml Diethylether versetzt, wobei das Produkt ausfällt. Man filtriert, wäscht mit Diethylether nach und trocknet dann im Hochvakuum. Es verbleiben 1.9 g eines weissen Produktes, das gemäss Elementaranalyse 2.20 % S enthält. Das Protonen NMR ist in Uebereinstimmung mit der nachfolgenden Struktur:

-[(CH₂-CHOH)ₐ-(CH₂-CHOCONHR)_{b}]ₙ-

wobei n etwa 10 und a:b = 20:1 ist; und R dem um das Isocyanat verminderten Rest der Titelverbindung aus Beispiel A7 entspricht.

### Beispiel B6, B7 und B8

In Analogie zu Beispiel B5 werden zwei Hydroxyalkyl-substituierte Polydimethylsiloxane (KF-6002/KF-6001) und ein Dextran mit dem Isocyanat aus Beispiel A7 umgesetzt. Die nachstehenden Parameter beschreiben diese Verbindungen. Die Ausbeuten betragen bei allen etwa 90 %. Der Schwefelgehalt dieser Verbindungen wird mittels Verbrennungsanalyse bestimmt (letzte Spalte der Tabelle).

| Isocyanat aus Beispiel A7 | OH-Makromer | Lösungsmittel | S-Gehalt (%) ber. / gef. |
|---|---|---|---|
| 0.5 g (0.94 mmol) | KF-6002, Shin-Etsu, JP 1.5g (0.63 Val OH/g) | THF | 1.50/1.38 |
| | | | |
| 0.5 g (0.94 mmol) | KF-6001, Shin-Etsu, JP 0.85g (1.1 mVal OH/g) | THF | 2.22/2.08 |
| | | | |
| 0.5 g (0.94 mmol) | Dextran 8, Serva G 2.3 g, MG ≈ 8 - 12000 | DMSO | 1.08/0.99 |

### Beispiel B9

In Analogie zu Beispiel B5 werden 3.23 g Collagen (Serva 17440, MG ≈ 80 000) während 12 Stunden in DMSO gelöst und dann mit 1.0 g (1.9 mmol) Isocyanat aus Beispiel A8 in 10 ml DMSO versetzt. Nach 72 Stunden Rühren bei RT wird das Reaktionsgemisch mit 500 ml Methanol verdünnt, worauf das Produkt ausfällt. Dies wird abfiltriert und mehrfach mit trockenem THF nachgewaschen. Dann wird im Hochvakuum (0.1 Pa, RT, 72 Stunden) getrocknet. Es verbleiben 2.8 g eines gelb-weissen Produktes, dessen IR-Spektrum und Protonen-NMR im Einklang mit der erwarteten Struktur steht.

### Beispiel B10

Herstellung eines Perfluoropolyethermakromers (Fomblin ZDOL TX 1000), beidseitig terminiert mit einem Isophorondiisocyanat-haltigen Photoinitiator

In einem 100 ml Kolbem mit Rückflusskühler, Thermometer, Rührer und Stickstoffeinleitungsrohr werden 4,46 g (0.01 mol) des reaktiven Photoinitiators (hergestellt gemäss Beispiel A1 der EP-A-632,329) in 10 g trockenem THF gelöst und mit 5,73 g (0.005 mol) Fomblin ZDOL TX 1000, Mw = 1146 (Ausimont SpA, Milano, Italien) vermischt. Hierzu gibt man 2 mg des Katalysators DBTDL und rührt 72 Stunden bei 40°C. Nach dieser Reaktionszeit kann IR-spektroskopisch kein unumgesetztes Isocyanat mehr nachgewiesen werden. Nach Abkühlen auf Raumtemperatur wird das Lösungsmittel durch Eindampfen am Rotationsverdampfer entfernt. Es wird ein hochviskoses, farbloses Oel erhalten, das noch im Hochvakuum von Spuren des Lösungsmittels befreit wird. Die Struktur des Produkts wird mittels ¹H-NMR-Spektrum verifiziert.

### C-Beispiele: Herstellung von linearen Triblock-Makromeren

### Beispiel C1:

In einem braunen Rundkolben mit Rückflusskühler, Rührer und Argon-Einleitungsrohr werden 800 mg (0.2 mmol) Makrophotoinitiator aus Beispiel B6 gemäss der EP-A-632,329 in 4 ml trockenem THF unter Argon gelöst. Hierzu gibt man 2,95 g (20 mmol) frisch destilliertes 2-Hydroxypropylmethacrylat (2-HPMA) und verrührt für 60 Minuten. Der Kolben mit seinem Inhalt wird dann in flüssigen Stickstoff für 10 Minuten getaucht und die gefrorene Lösung für 15 Minuten unter reduziertem Druck (0.004 mbar) entgast. Das Vakuum wird mit Argon entspannt und die Lösung auf Raumtemperatur erwärmt und 15 Minuten mit Argon begast. Nach Filtration durch Filter mit 0,45 µm Porengrösse werden saubere Polypropylen - Formen unter Stickstoff mit dieser Lösung gefüllt (etwa 200 µl Lösung pro Form), verschlossen und für 20 Minuten mit UV-Licht (12 mW/cm²) bestrahlt. Die Formen mit der hochviskosen Polymerlösung werden dann im Trockenschrank bei 40°C von THF befreit. Es verbleiben klare, durchsichtige Scheibchen (Disks), die in Ethanol löslich sind. Das Mw des Triblock-Makromeren gemäss GPC - Analyse ist 18700. Dabei fällt auf, dass das GPC ausschliesslich die Bildung des Triblock-Copolymeren zeigt und dass dabei kein Homopolymer des eingesetzten Vinylmonomeren als Nebenprodukt beobachtet wird.

### Beispiel C2:

In Analogie zu Beispiel C1 werden 1,96 g (0,5 mmol) Siloxan-Makrophotoinitiator B6 gemäss EP-A-632329 in 4 ml trockenem THF unter Inertgasatmosphäre gelöst. Hierzu gibt man 720 mg (5 mmol) frisch destilliertes 2-HPMA und rührt für 60 Minuten. Die nachfolgende Probenpräparation ist gleich wie in Beispiel C1. Das Mw von diesem Triblock-Makromer entspricht gemäss GPC - Analyse 7800. Dabei fällt auf, dass das GPC ausschliesslich die Bildung des Triblock-Copolymeren zeigt und dass dabei kein Homopolymer des eingesetzten Vinylmonomeren als Nebenprodukt beobachtet wird.

### Beispiele C3 bis C9:

Entsprechend Beispiel C1 werden weitere Triblock-Makromere mit unterschiedlichen Makrophotoinitiatoren (siehe B-Beispiele) sowie mit unterschiedlicher Art und Zusammensetzung der Comonomere hergestellt. Die nachfolgende tabellarische Aufstellung enthält die wichtigsten Parameter (die Abkürzungen haben die folgenden Bedeutungen: HEMA = 2-Hydroxyethylmethacrylat, 2-HPMA = 2-Hydroxypropylmethacrylat, PME 400 = Polyethylenglykol(Mw 400)methacrylat, NVP = N-Vinyl-2-pyrrolidon):

| Beispiel C3: | |
|---|---|
| Makroinitiator ("MI"): | B10 gemäss EP-A-632329, |
| Comonomer: | HEMA, |
| Verhältnis MI/Comonomer (mol/mol) | 1:13, |
| Lösungsmittel, Konzentration: | Ethanol, 33 %, |
| Bestrahlungszeit (Minuten): | 20, |
| Mw des Produktes gemäss GPC: | 3547. |

| Beispiel C4: | |
|---|---|
| Makroinitiator ("MI"): | B10 gemäss EP-A-632329, |
| Comonomer: | PME 400, |
| Verhältnis MI/Comonomer (mol/mol) | 1:100, |
| Lösungsmittel, Konzentration: | THF, 50 %, |
| Bestrahlungszeit (Minuten): | 25, |
| Mw des Produktes gemäss GPC: | 39620. |

| Beispiel C5 | |
|---|---|
| Makroinitiator ("MI"): | B6 gemäss EP-A-632329, |
| Comonomer: | HEMA, |
| Verhältnis MI/Comonomer (mol/mol) | 1:10, |
| Lösungsmittel, Konzentration: | THF, 50 %, |
| Bestrahlungszeit (Minuten): | 25, |
| Mw des Produktes gemäss GPC: | 13590. |

| Beispiel C6: | |
|---|---|
| Makroinitiator ("MI"): | B2 gemäss EP-A-632329, |
| Comonomer: | 2-HPMA, |
| Verhältnis MI/Comonomer (mol/mol) | 1:35, |
| Lösungsmittel, Konzentration: | THF, 35 %, |
| Bestrahlungszeit (Minuten): | 20, |
| Mw des Produktes gemäss GPC: | 26600. |

| Beispiel C7: | |
|---|---|
| Makroinitiator ("MI"): | B2 gemäss EP-A-632329, |
| Comonomer: | PME 400, |
| Verhältnis MI/Comonomer (mol/mol) | 1:100, |
| Lösungsmittel, Konzentration: | THF, 50 %, |
| Bestrahlungszeit (Minuten): | 25, |
| Mw des Produktes gemäss GPC: | 97651. |

| Beispiel C8: | |
|---|---|
| Makroinitiator ("MI"): | B10, |
| Comonomer: | 2-HPMA, |
| Verhältnis MI/Comonomer (mol/mol) | 1:6, |
| Lösungsmittel, Konzentration: | THF, 30 %, |
| Bestrahlungszeit (Minuten): | 20, |
| Mw des Produktes gemäss GPC: | 1900. |

| Beispiel C9: | |
|---|---|
| Makroinitiator ("MI"): | B4 |
| Comonomer: | NVP, |
| Verhältnis MI/Comonomer (mol/mol) | 1:10, |
| Lösungsmittel, Konzentration: | Ethanol, 30 %, |
| Bestrahlungszeit (Minuten): | 20, |
| Mw des Produktes gemäss GPC: | 2960. |

### Beispiel C10:

2,0 g Makroinitiator aus Beispiel B3 werden in 3 ml trockenem THF unter Stickstoff gelöst. 2 g dieser Lösung werden mit 0,9 g (8 mmol) frisch destilliertem NVP vermischt und für 30 Minuten mit Stickstoff begast. Dann werden saubere Polypropylen-Formen unter Stickstoff mit dieser Lösung gefüllt (etwa 200 µl Lösung pro Form), verschlossen und für 10 Minuten mit UV-Licht (12,5 mW/cm²) bestrahlt. Die Formen mit der hochviskosen Polymerlösung werden dann im Trockenschrank bei 40°C vom THF befreit. Es verbleiben klare, leicht gelbe Scheiben (Disks), die in Ethanol löslich sind. Das Mw beträgt gemäss GPC etwa 5800.

### Beispiel C11:

In Analogie zu Beispiel C10 werden transparente, leicht opake Disks hergestellt aus einem Gemisch von 2,0 g Makroinitiator aus Beispiel B3 und 0,9 g (8,15 mmol) Dimethylacrylamid (DMA). Mw dieses Triblock-Makromeren entspricht gemäss GPC 5560.

### D-Beispiele: Herstellung von vernetzten Disks oder Linsen

### Beispiel D1:

2,0 g (0,5 mmol) Makrophotoinitiator gemäss B6 von EP-A-632329 werden in einem 20 ml Rundkolben unter Stickstoffatmosphäre in 3 ml Ethanol gelöst. Nach Zugabe von 2,6 g (0,02 mol) HEMA und 0,4 g (8%) Ethylenglykoldimethacrylat (EGDMA) als Vernetzer wird die Reaktionsmischung während 2 Stunden unter Stickstoff gerührt. Die Lösung wird dann mit flüssigem Stickstoff eingefroren und im Hochvakuum (0,004 mbar) für 15 Minuten entgast. Die nachfolgende Probenpräparation sowie die Polymerisation werden innerhalb einer Glove Box unter Sauerstoffausschluss durchgeführt.-Die Reaktionsmischung wird erst 15 Minuten gerührt, dann mikrofiltriert (0,45 µm Filter) und anschliessend in Propylenformen eingefüllt. Es können sowohl Kontaktlinsenformen als auch Formen für runde Scheibchen (Disks) verwendet werden. Die geschlossenen Formen werden in einem dafür eingerichteten UV-Ofen für 20 Minuten bei einer UV-Intensität von 12,5 mW/cm² bestrahlt. Die fertigen Disks oder Linsen werden in Ethanol 24 Stunden extrahiert und im Hochvakuum bei 40°C und 10⁻³ Torr getrocknet. Die Disks zeigen einen E-Modul von 2.1 MPa und eine Sauerstoffdurchlässigkeit von 98 Barrers.

### Beispiel D2:

In Analogie zu Beispiel D1 werden Linsen und Disks hergestellt aus 2,0 g (0,5 mmol) Makroinitiator von Beispiel B6 gemäss EP-A-632329, 1,3 g (10 mmol) HEMA, 0,23 g (6,5 %) EGDMA und 3 ml Isopropanol.

### Beispiel D3:

In Analogie zu Beispiel D1 werden Linsen hergestellt aus 1 g (0,25 mmol) Makroinitiator von Beispiel B6 gemäss EP-A-632329, 1 g (10 mmol) DMA, 0,2 g (9 %) EGDMA und 2,2 g Isopropanol.

### Beispiel D4:

In Analogie zu Beispiel D1 werden Linsen hergestellt aus 3,45 g Makroinitiator aus Beispiel B1, 5,95 g 3-[Tris(trimethylsiloxy)-silyl]propylmethacrylat (TRIS), 0.6 g NVP und 0,5 g (4,75 %) EGDMA, wobei TRIS und NVP als Lösungsmittel für den entsprechenden Makroinitiator dienen.

### Beispiel D5:

In Analogie zu Beispiel D1 werden Disks hergestellt aus 3,92 g (1 mmol) Makroinitiator aus Beispiel B3, 3,92 g (8 mmol) PME 400, und 0,5 g (6 %) EGDMA. THF (5 ml) wird als Lösungsmittel verwendet.

### Beispiel D6:

In Analogie zu Beispiel D1 werden Disks hergestellt aus 1,0 g (0,5 mmol) Makroinitiator aus Beispiel B10, 0,28 g (2,5 mmol) NVP, 0,1 g (7,2 %) EGDMA und 1 g THF.

Die nachfolgende Uebersicht zeigt die Eigenschaften der resultierenden Linsen und Disks:

| Beispiel | Wasseraufnahme (%) | E-Modul (MPa) | Bruchdehnung (%) |
|---|---|---|---|
| D2 | 25,2 | 2,8 | 140 |
| D3 | 39,1 | 3,6 | 69 |
| D4 | 5,2 | 0,1 | 97.4 |
| D5 | 41,1 | 0,8 | 144 |
| D6 | 4,0 | 3,0 | 74 |

### Beispiel D7:

2,5 g (0,3 mmol) Makroinitiator auf Beispiel B5 gemäss der EP-A-632329 werden in 4 ml trockenem Ethanol unter Stickstoff gelöst. Hierzu gibt man 2,4 g (0,018 mol) frisch destilliertes HEMA und 0,4 g (7,5 %) des Vernetzers EGDMA. Die Reaktionsmischung wird dann während 30 Minuten unter Stickstoff gerührt und anschliessend für 15 Minuten mit Stickstoff begast. Dann wird die Lösung in eine Flasche filtriert (Porengrösse 0,45 µm). Unter Stickstoff werden saubere Polypropylen-Formen mit Lösung gefüllt (200 µl pro Form), die Formen werden geschlossen und für 25 Minuten mit UV-Licht bestrahlt. Die Formen werden geöffnet und die Formhälften, enthaltend die Linsen, in ein Ethanolbad eingelegt, wobei die Linsen sich von den Formhälften lösen. Die Linsen werden dann noch 24 Stunden in Ethanol extrahiert und anschliessend im Hochvakuum (10⁻⁴ Torr) getrocknet. Nach Autoklavierung werden die Linsen analysiert. Die Linsen weisen eine Sauerstoffdurchlässigkeit von 91,3 Barrers auf und Kontaktwinkel von 110° (advancing) und 102° (receding).

### Beispiel D8

0.16 g Makrophotoinitiator aus Beispiel B5 werden unter Stickstoff in 0.82 g einer Lösung von N-Methylpyrrolidon (NMP) in DMSO (70:12) gelöst. Hierzu gibt man 20 µg des Vernetzers EGDMA und begast dann für 20 Minuten mit Stickstoff. Dann wird die Lösung in eine Flasche filtriert (Teflonfilter mit Porengrösse 0.45 µm). Unter Stickstoff werden saubere PP-Formen mit dieser Lösung gefüllt (180 - 200 µl pro Form), die Formen werden geschlossen und für 30 Minuten mit UV-Licht bestrahlt (12 mW/cm²). Die Formen werden geöffnet und die Formhälften, enthaltend die Linsen, in ein Ethanolbad eingelegt, wobei die durchsichtigen, schwach gelben Linsen sich von den Formhälften lösen. Die Linsen werden dann noch 24 Stunden in Ethanol extrahiert und anschliessend im Vakuum getrocknet.

### Beispiel D9

In Analogie zu Beispiel D8 werden Kontaktlinsen hergestellt aus 0.1 g Makrophotoinitiator aus Beispiel B5, 0.5 g DMSO, 0.4 g NVP und 20 µg EGDMA.

### Beispiel D10

0.25 g Makrophotoinitiator aus Beispiel B8 werden in 0.5 g trockenem DMSO unter Stickstoff gelöst. Hierzu gibt man 0.25 g HEMA und 20 µg des Vernetzers EGDMA. Anschliessend wird für 30 Minuten mit Stickstoff begast. Dann wird die Lösung filtriert (Porengrösse 0.45 µm) und unter Stickstoff in saubere PP-Formen gefüllt. Es wird wie in Beispiel D8 bestrahlt und aufgearbeitet.

Die nachfolgende Tabelle gibt Aufschluss über die Parameter der so hergestellten Kontaktlinsen (wobei "Strain" auch "Bruchdehnung" genannt wird):

| Beispiel | Wasseraufnahme(%) | Strain (%) | E-Modu (MPa) |
|---|---|---|---|
| D8 | 178 | 490 | 0.04 |
| D9 | 441 | 73 | 0.24 |
| D10 | 19 | 67 | 0.52 |

### Beispiel D11:

Die Polymerisation wird analog zu Beispiel D8 durchgeführt mit der folgenden Zusammensetzung: Makroinitiator von Beispiel B4 (20%), DMA (20%), TRIS (5%), EGDMA (5%). Anstelle von NMP und DMSO wird die Polymerisation in Ethanol (40 %) durchgeführt. Die Linsen zeigen eine Wasserufnahme von 160 % und einen E-Modul von 0,34 MPa.

### Beispiel D12:

2,5 g des in Beispiel C5 beschriebenen Triblockcopolymeren - bestehend aus zentralem Polysiloxanblock und zwei terminalen Poly-HEMA - Blöcken - werden unter trockenem Stickstoff in 3,5 ml trockenem THF gelöst und mit 0,08 g 2-Isocyanatoethylmethacrylat (IEM) sowie 5 mg Dibutylzinndilaurat versetzt. Die Mischung wird 24 Stunden bei 40°C gerührt, bis alles IEM reagiert hat (IR-Spektrum). Dann wird durch Zusatz von 0,25 g DMA sowie von 150 mg Irgacure 184 als Photoinitiator eine Kontaktlinsenformulierung zubereitet. Gemäss Beispiel D1 wird diese Formulierung zur Herstellung von weichen Kontaktlinsen verwendet. Nach der Autoklavierung zeigen die so erhaltenen Kontaktlinsen eine Sauerstoffpermeabilität von 87 barrers.

## Patentansprüche

1. Polymer, bei dem es sich um das Polymerisationsprodukt eines polymerisationsfähigen Gemisches handelt, das die folgenden Bestandteile enthält:
a) ein Makromer der Formel C 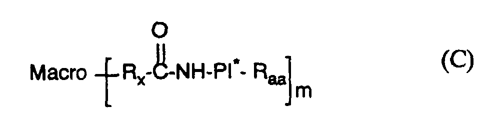 worin Macro für einen m-wertigen Rest eines Makromeren steht, von dem die Anzahl von m Gruppen Rₓ-H entfernt ist,
Rₓ unabhängig voneinander für eine Bindung, -O-, -NR_{N}- oder -S- steht, worin R_{N} für Wasserstoff oder Niederalkyl steht,
PI* für einen zweiwertigen Rest eines Photoinitiators steht,
Rₐₐ für den Teil eines Photoinititors steht, der bei einer Aufspaltung des Photoinitiators das weniger reaktive Radikal bildet, und
m für eine ganze Zahl von 1 bis 100 steht,
b) ein copolymerisierbares Vinylmonomer und
c) einen Vernetzer.

2. Polymer gemäss Anspruch 1, dadurch gekennzeichnet, dass der Vernetzer eine copolymerisierbare oligoolefinische Verbindung ist.

3. Polymer gemäss Anspruch 1, dadurch gekennzeichnet, dass der Vernetzer eine oligofunktionelle Verbindung ist, die mit reaktiven Gruppen coreaktiv ist, die im copolymerisierbaren Vinylmonomer enthalten sind.

4. Polymer gemäss Anspruch 1, dadurch gekennzeichnet, dass zumindest einige der copolymerisierbaren und bereits copolymerisierten Vinylmonomere in einem der Polymerisation nachgeschalteten Schritt so modifiziert werden, dass sich die modifizierten copolymerisierten Vinylmonomere vernetzen lassen.

5. Segmentiertes Copolymer der Formel I, 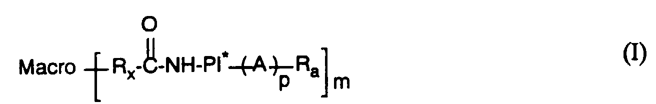 worin Macro für einen m-wertigen Rest eines Makromeren steht, von dem die Anzahl von m Gruppen Rₓ-H entfernt ist,
Rₓ unabhängig voneinander für eine Bindung, -O-, -NR_{N}- oder -S- steht, worin R_{N} für Wasserstoff oder Niederalkyl steht,
PI* für einen zweiwertigen Rest eines Photoinitiators steht,
A für einen bivalenten, substituierten 1,2-Ethylenrest steht, der sich von einem copolymerisierbaren Vinylmonomer dadurch ableitet, dass die Vinyl-Doppelbindung durch eine Einfachbindung ersetzt ist,
jedes Rₐ unabhängig voneinander für eine einwertige Gruppe steht, die geeignet ist, als Kettenabbrecher einer Polymerisation zu dienen,
p unabhängig von m für eine ganze Zahl von 3 bis 500 steht und
m für eine ganze Zahl von 1 bis 100 steht.

6. Copolymer der Formel I gemäss Anspruch 5, worin m für zwei steht und die beiden Gruppen, die an den Bestandteil "Macro" gebunden sind, terminal an "Macro" gebunden sind, so dass es sich um ein Triblockcopolymer handelt.

7. Copolymer der Formel I gemäss Anspruch 5, worin die Anzahl von m Gruppen, die an den Bestandteil "Macro" gebunden sind, ausschliesslich pendent an "Macro" gebunden sind, so dass es sich um ein Kammpolymer handelt.

8. Copolymer der Formel I gemäss Anspruch 5, worin "Macro" für ein cyclisches Makromer steht und die Anzahl von m Gruppen, die an den Bestandteil "Macro" gebunden sind, pendent an "Macro" gebunden sind, so dass es sich um ein Stempolymer handelt.

9. Copolymer gemäss Anspruch 6, worin Macro für den Rest eines Polysiloxans oder eines fluorierten Polyethers steht und der Teil A von einem hydrophilen Vinylmonomer abgeleitet ist, das eine reaktive Gruppe aufweist.

10. Copolymer gemäss Anspruch 9, worin es sich bei der reaktiven Gruppe um Hydroxy oder Isocyanato handelt.

11. Copolymer gemäss Anspruch 10, worin das Vinylmonomer ein Hydroxy-niederalkyl(meth)acrylat oder ein Isocyanato-niederalkyl(meth)acrylat ist.

12. Copolymer gemäss Anspruch 6, worin Macro für den Rest eines Polysiloxans oder eines fluorierten Polyethers steht und der Teil A von einem hydrophilen Vinylmonomer abgeleitet ist, das keine reaktive Gruppe aufweist.

13. Copolymer gemäss Anspruch 12, worin das Vinylmonomer ein Vinyllactam ist.

14. Copolymer gemäss Anspruch 6, worin Macro für den Rest eines hydrophilen Macromeren steht und der Teil A von einem hydrophoben Vinylmonomer abgeleitet ist.

15. Formkörper enthaltend ein Polymer gemäss Anspruch 1.

16. Formkörper gemäss Anspruch 15, bei dem es sich um eine Kontaktlinse handelt.

17. Verwendung eines Polymeren gemäss Anspruch 1 zur Herstellung eines Formkörpers.

18. Verwendung gemäss Anspruch 17, wobei es sich bei dem Formkörper um eine Kontaktlinse handelt.

19. Verfahren zur Herstellung einer Kontaktlinse, dadurch gekennzeichnet, dass ein Polymer gemäss Anspruch 1 in Form einer Kontaktlinse hergestellt oder in die Form einer Kontaktlinse übergeführt wird.

20. Verfahren zur Herstellung eines Polymeren gemäss Anspruch 1, dadurch gekennzeichnet, dass die Komponenten a), b) und c) gemäss Anspruch 1 copolymerisiert und vernetzt werden,
oder dadurch dass die Komponenten a) und b) gemäss Anspruch 1 copolymerisiert werden und die erhaltene Verbindung der Formel I, gemäss Anspruch 5, durch Umsetzung mit einer oligofunktionellen Verbindung, die Gruppen enthält, die mit im -(A)ₚ- Teil enthaltenen reaktiven Gruppen coreaktiv sind, vernetzt wird,
oder dadurch dass die Komponenten a) und b) gemäss Anspruch 1 copolymerisiert werden, zumindest einige der copolymerisierbaren und bereits copolymerisierten Vinylmonomere in einem der Polymerisation nachgeschalteten Schritt so modifiziert werden, dass sich die modifizierten copolymerisierten Vinylmonomere vernetzen lassen und dass sodann vernetzt wird.

21. Verfahren zur Herstellung eines Copolymeren der Formel I gemäss Anspruch 5, dadurch gekennzeichnet, dass ein Makromer der Formel C, wie in Anspruch 1 definiert, mit p Moläquivalenten eines Vinylmonomeren umgesetzt wird.

## Claims

1. A polymer, which is the polymerisation product of a polymerisable mixture that comprises the following components:
a) a macromer of formula C 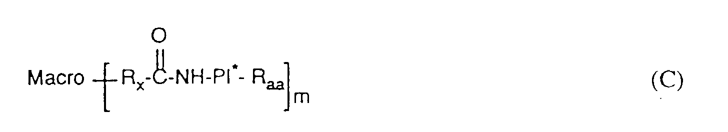 wherein Macro is an m-valent radical of a macromer from which the number m of groups Rₓ-H has been removed,
each Rₓ, independently of the others, is a bond, -O-, -NR_{N}- or -S- wherein R_{N} is hydrogen or lower alkyl,
PI* is a bivalent radical of a photoinitiator,
Rₐₐ is the moiety of a photoinitiator that forms the less reactive free radical on cleavage of the photoinitiator, and
m is an integer from 1 to 100,
b) a copolymerisable vinyl monomer and
c) a crosslinker.

2. A polymer according to claim 1, wherein the crosslinker is a copolymerisable oligo-olefinic compound.

3. A polymer according to claim 1, wherein the crosslinker is an oligofunctional compound that is co-reactive with reactive groups present in the copolymerisable vinyl monomer.

4. A polymer according to claim 1, wherein at least some of the copolymerisable and already copolymerised vinyl monomers are so modified in a step subsequent to the polymerisation that the modified copolymerised vinyl monomers can be crosslinked.

5. A segmented copolymer of formula I 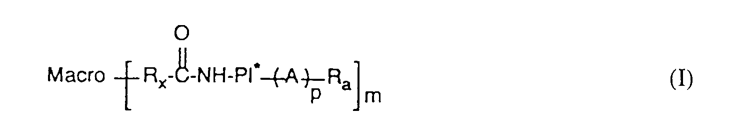 wherein Macro is an m-valent radical of a macromer from which the number m of groups Rₓ-H has been removed,
each Rₓ, independently of the others, is a bond, -O-, -NR_{N}- or -S- wherein R_{N} is hydrogen or lower alkyl,
PI* is a bivalent radical of a photoinitiator,
A is a substituted bivalent 1,2-ethylene radical derivable from a copolymerisable vinyl monomer by replacing the vinyl double bond by a single bond,
each Rₐ, independently of the others, is a monovalent group that is suitable to act as a polymerisation chain-reaction terminator, and
p, independently of m, is an integer from 3 to 500, and
m is an integer from 1 to 100.

6. A copolymer of formula I according to claim 5, wherein m is 2 and the two groups bonded to the component "Macro" are terminally bonded to "Macro" so that it is a tri-block copolymer.

7. A copolymer of formula I according to claim 5, wherein the number m of groups that are bonded to the component "Macro" are bonded exclusively pendantly to "Macro" so that it is a comb polymer.

8. A copolymer of formula I according to claim 5, wherein "Macro" is a cyclic macromer and the number m of groups that are bonded to the component "Macro" are bonded pendantly to "Macro" so that it is a star polymer.

9. A copolymer according to claim 6, wherein Macro is the radical of a polysiloxane or of a fluorinated polyether and the moiety A is derived from a hydrophilic vinyl monomer having a reactive group.

10. A copolymer according to claim 9, wherein the reactive group is hydroxy or isocyanato.

11. A copolymer according to claim 10, wherein the vinyl monomer is a hydroxy-lower alkyl (meth)acrylate or an isocyanato-lower alkyl (meth)acrylate.

12. A copolymer according to claim 6, wherein Macro is the radical of a polysiloxane or of a fluorinated polyether and the moiety A is derived from a hydrophilic vinyl monomer having no reactive group.

13. A copolymer according to claim 12, wherein the vinyl monomer is a vinyl-lactam.

14. A copolymer according to claim 6, wherein Macro is the radical of a hydrophilic macromer and the moiety A is derived from a hydrophobic vinyl monomer.

15. A moulding comprising a polymer according to claim 1.

16. A moulding according to claim 15 which is a contact lens.

17. The use of a polymer according to claim 1 in the production of a moulding.

18. Use according to claim 17, wherein the moulding is a contact lens.

19. A process for the production of a contact lens, wherein a polymer according to claim 1 is produced in the form of a contact lens or is converted into the form of a contact lens.

20. A process for the preparation of a polymer according to claim 1, wherein
the components a), b) and c) according to claim 1 are copolymerised and crosslinked. or the components a) and b) according to claim 1 are copolymerised and the resulting compound of formula I, according to claim 5, is crosslinked by reaction with an oligofunctional compound containing groups that are co-reactive with reactive groups present in the -(A)ₚ- moiety,
or the components a) and b) according to claim 1 are copolymerised, at least some of the copolymerisable and already copolymerised vinyl monomers are so modified in a step subsequent to the polymerisation that the modified copolymerised vinyl monomers can be crosslinked, and then crosslinking is carried out.

21. A process for the preparation of a copolymer of formula I according to claim 5, wherein a macromer of formula C, as defined in claim 1, is reacted with p mol equivalents of a vinyl monomer.

## Revendications

1. Un polymère qui est le produit de polymérisation d'un mélange polymérisable qui comprend les composants suivants:
a) un macromère de formule C 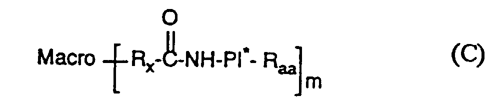 où macro signifie un reste m-valent d'un macromère dont on a éliminé le nombre m de groupes Rₓ-H,
chaque Rₓ indépendamment l'un de l'autre, signifie une liaison, -O-, -NR_{N}- ou -S-, où R_{N} signifie l'hydrogène ou un groupe alkyle inférieur,
PI* signifie un reste bivalent d'un photoinitiateur,
Rₐₐ signifie le reste d'un photoinitiateur, qui forme le radical le moins réactif lors du clivage du photoinitiateur, et
m signifie un nombre entier de 1 à 100,
b) un monomère vinylique copolymérisable, et
c) un agent de réticulation.

2. Un polymère selon la revendication 1, caractérisé en ce que l'agent de réticulation est un composé oligooléfinique copolymérisable.

3. Un polymère selon la revendication 1, caractérisé en ce que l'agent de réticulation est un composé oligofonctionnel, qui est coréactif avec des groupes réactifs qui sont contenus dans le monomère vinylique copolymérisable.

4. Un polymère selon la revendication 1, caractérisé en ce qu'au moins plusieurs des monomères vinyliques copolymérisables et déjà copolymérisés sont modifiés dans une étape après la polymérisation, de sorte que les monomères vinyliques copolymérisés modifiés puissent être réticulés.

5. Un copolymère segmenté de formule I 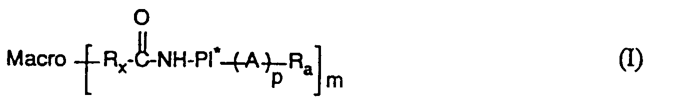 où macro signifie un reste m-valent d'un macromère dont on a éliminé le nombre m de groupes Rₓ-H,
chaque Rₓ indépendamment l'un de l'autre, signifie une liaison, -O-, -NR_{N}- ou -S-, où R_{N} signifie l'hydrogène ou un groupe alkyle inférieur,
PI* signifie un reste bivalent d'un photoinitiateur,
A signifie un reste 1,2-éthylène bivalent substitué, qui est dérivé d'un monomère vinylique copolymérisable, de sorte que la double liaison vinylique est remplacée par une simple liaison,
chaque Rₐ indépendamment l'un de l'autre, signifie un groupe monovalent qui est approprié pour servir d'interrupteur de la chaîne d'une polymérisation,
p indépendamment de m, signifie un nombre entier de 3 à 500, et
m signifie un nombre entier de 1 à 100.

6. Un copolymère de formule I selon la revendication 5, où m signifie 2 et les deux groupes qui sont liés au composant "macro", sont liés à l'extrêmité au "macro", pour former un copolymère triséquencé.

7. Un copolymère de formule I selon la revendication 5, où le nombre m de groupes qui sont liés au composant "macro", sont liés exclusivement latéralement au "macro", pour former un polymère peigné.

8. Un copolymère de formule I selon la revendication 5, où "macro" signifie un macromère cyclique et le nombre m de groupes qui sont liés au composant "macro", sont liés latéralement au "macro", pour former un polymère étoilé.

9. Un copolymère selon la revendication 6, où macro signifie le reste d'un polysiloxane ou d'un polyéther fluoré et le reste A est dérivé d'un monomère vinylique hydrophile qui comporte un groupe réactif.

10. Un copolymère selon la revendication 9, où le groupe réactif est un groupe hydroxy ou isocyanate.

11. Un copolymère selon la revendication 10, où le monomère vinylique est un (méth)acrylate d'hydroxy-alkyle inférieur ou un (méth)acrylate d'isocyanatealkyle inférieur.

12. Un copolymère selon la revendication 6, où macro signifie le reste d'un polysiloxane ou d'un polyéther fluoré et le reste A est dérivé d'un monomère vinylique hydrophile qui ne comporte pas de groupe réactif.

13. Un copolymère selon la revendication 12, où le monomère vinylique est un vinyllactame.

14. Un copolymère selon la revendication 6, où macro signifie le reste d'un macromère hydrophile et le reste A est dérivé d'un monomère vinylique hydrophobe.

15. Un moulage contenant un polymère selon la revendication 1.

16. Un moulage selon la revendication 15, qui est une lentille de contact.

17. Utilisation d'un polymère selon la revendication 1, pour la préparation d'un moulage.

18. Utilisation selon la revendication 17, où le moulage est une lentille de contact.

19. Un procédé de préparation d'une lentille de contact, caractérisé en ce qu'on prépare un polymère selon la revendication 1 sous forme d'une lentille de contact ou on le transforme sous forme d'une lentille de contact.

20. Un procédé de préparation d'un polymère selon la revendication 1, caractérisé en ce que les composants a), b) et c) selon la revendication 1 sont copolymérisés et réticulés,
ou bien en ce que les composants a) et b) selon la revendication 1 sont copolymérisés et le composé obtenu de formule I, selon la revendication 5, est réticulé par réaction avec un composé oligofonctionnel qui contient des groupes qui sont coréactifs avec les groupes réactifs contenus dans le reste -(A)ₚ,
ou bien en ce que les composants a) et b) selon la revendication 1 sont copolymérisés, au moins certains des monomères vinyliques copolymérisables et déjà copolymérisés sont modifiés dans une étape après la polymérisation, de sorte que les monomères vinyliques copolymérisés modifiés puissent être réticulés et en ce que la réticulation est ensuite effectuée.

21. Un procédé de préparation d'un copolymère de formule I selon la revendication 5, caractérisé en ce qu'un macromère de formule C, tel que défini à la revendication 1, est mis à réagir avec p équivalents molaires d'un monomère vinylique.
